# EUROPEAN PATENT SPECIFICATION

(11) **EP 1 180 167 B1**
(45) Date of publication and mention of the grant of the patent: **21.02.2007**
(21) Application number: 00935391.3
(22) Date of filing: 02.06.2000
(51) Int. Cl.: C12Q 1/68, C12N 15/86, C12N 7/01

(54) **IN VIVO SELECTION METHOD FOR DETERMINING INHIBITORY RNA MOLECULES**
INVIVO SELEKTIONSVERFAHREN ZUR BESTIMMUNG VON INHIBIERENDEN RNA MOLEKÜLEN
PROCEDE DE SELECTION IN VIVO PERMETTANT DE DETERMINER LES MOLECULES INHIBITRICES D'ARN

(30) Priority: 03.06.1999 GB 9912965
(43) Date of publication of application: 20.02.2002
(73) Proprietor: Oxford Biomedica (UK) Limited, Oxford 0X4 4GA (GB)
(72) Inventor: Mitrophanous, Kyri, Oxford OX1 4TR (GB); Kim, Narry Howard Hughes Medical Institute, 326 Clinical Research Building 415 Curie (US); Kotsopoulou, Ekaterini, Boston, MA 02115 (US)
(74) Representative: Holliday, Louise Caroline
(86) International application number: PCT/GB2000/002136
(87) International publication number: WO 2000/075370

(56) References cited:
- WO-A-92/01806
- WO-A-95/29254
- WO-A-98/06440
- WO-A-99/27135
- DE-C- 4 424 762
- US-A- 5 624 824
- US-A- 5 869 248
- GUERRIER-TAKADA C ET AL: "PHENOTYPIC CONVERSION OF DRUG-RESISTANT BACTERIA TO DRUG SENSITIVITY" PROCEEDINGS OF THE NATIONAL ACADEMY OF SCIENCES OF USA,US,NATIONAL ACADEMY OF SCIENCE. WASHINGTON, vol. 94, no. 16, 5 August 1997 (1997-08-05), pages 8468-8472, XP002052175 ISSN: 0027-8424
- YUAN Y AND ALTMAN S: "Selection of guide sequences that direct efficient cleavage of mRNA by human ribonuclease P" SCIENCE,AMERICAN ASSOCIATION FOR THE ADVANCEMENT OF SCIENCE,,US, vol. 263, 4 March 1994 (1994-03-04), pages 1269-1273, XP002104825 ISSN: 0036-8075

## Description

### Field of the Invention

The present invention relates to an *in vivo* method for selecting optimal inhibitory RNA molecules.

### Background to the Invention

There is considerable interest in the use of inhibitory RNA molecules to inhibit the transcription and/or translation of target nucleic acid sequences. An inhibitory RNA molecule may be capable of binding to and effecting the cleavage, directly or indirectly, of a nucleotide sequence, or its transcription product. Examples include ribozymes (which are capable of direct cleavage of the target sequence), external guide sequences (EGSs) and anti-sense or sense RNA (which are capable of causing cleavage by other factors).

Unfortunately the application of such technology has encountered difficulties due to the complex secondary structure of mRNA. It is currently impossible to predict the location of sites within mRNA that are open or accessible other than by trying to target every location.

One such approach used to search for these open areas is the *in vitro* 'Oligonucleotide Arrays' method described by Southern (1997) and Milner (1997). However, these methods involve the time consuming generation of unique arrays for each particular target mRNA. In addition the formation of heteroduplexes is carried out in non-physiological conditions. This is especially problematic when one considers that mRNA has a number of proteins associated at both the 5' and 3' ends.

It would therefore be advantagous to use an *'in vivo'* selection method to discover open mRNA sites in their normal physiological context. In addition the method should be generally useful in that all RNA molecules could be scanned in this way without having to resort to particular selection methods each time.

DE 4424762 C (MAX PLANCK GESELLSCHAFT, 27 July 1995) discloses a plurality of first nucleotide sequences having the following combination of features(abstract; claims 1-3): they encode a gene product capable of binding to and effecting the cleavage of a nucleotide sequence or the transcription product thereof, and a region of the sequence required for binding is heterogeneous within the plurality of sequences. These nucleotide sequences can be used in a selection system suitable for in vivo selecting of inhibitory RNA molecules.

### Summary of the Invention

It is therefore an object of the present invention to provide an *in vivo* selection method for identifying inhibitory RNA molecules that target efficiently a given RNA sequence.

The present inventors have shown that it is possible to select inhibitory RNA molecules (such as ribozymes) *in vivo* using cells which express a target sequence operably linked to a detectable marker, such that the target sequence and detectable marker are expressed as a contiguous RNA molecule in the cells.

The target sequence/detectable marker mRNA will contain the normal 5' cap and the 3' poly A tail. If the 5' cap or the 3' poly A tail are removed (for example following cleavage of the target sequence by an inhibitory RNA molecule) the mRNA will become unstable and will be degraded. This will prevent translation of the detectable marker gene.

If, for example, the detectable marker is an enzyme capable of converting a prodrug into a cytotoxic compound, cleavage of the target sequence will render the cell insensitive to the prodrug.

Cells expressing the detectable marker/target gene may be used to screen a large number of inhibitory RNA molecules. In particular, the present inventors have used such cells to screen a library of ribozyme-expressing vectors with each ribozyme having a different specificity region. Cells which are resistant to the prodrug will contain an active ribozyme, the sequence of which can then be determined using techniques such as the polymerase chain reaction (PCR).

Accordingly the present invention provides a selection system suitable for use *in vivo,* the system comprising:
(i) a plurality of first nucleotide sequences encoding a gene product capable of binding to and effecting the cleavage, directly or indirectly, of a nucleotide sequence, or a transcription product thereof;
   wherein a region of the first nucleotide sequence required for binding to the nucleotide sequence is heterogeneous within the plurality of first nucleotide sequences; and
(ii) a second nucleotide sequence comprising
   (a) a coding region encoding a detectable marker operably linked to sequences required for mRNA stability and/or translation: and
   (b) a third nucleotide sequence positioned between the coding region and at least one of the sequences required for mRNA stability and/or translation:
wherein (a) and (b) are operably linked to a regulatory sequence capable of directing expression of (a) and (b) as a contiguous RNA molecule in a host cell.

The present invention also provides a vector system comprising:
(i) a plurality of vectors, each vector independently comprising
   a first nucleotide sequence encoding a gene product capable of binding to and effecting the cleavage, directly or indirectly, of a nucleotide sequence, or a transcription product thereof;
   wherein a region of the first nucleotide sequence required for binding to the nucleotide sequence is heterogeneous within the plurality of vectors; and
(ii) a second nucleotide sequence comprising
   (a) a coding region encoding a detectable marker operably linked to sequences required for mRNA stability and/or translation; and
   (b) a third nucleotide sequence positioned between the coding region and at least one of the sequences required for mRNA stability and/or translation;
wherein (a) and (b) are operably linked to a regulatory sequence capable of directing expression of (a) and (b) as a contiguous RNA molecule in a host cell,
wherein the second nucleotide sequence is present in the plurality of vectors or as part of a separate vector.

Preferably the gene product is selected from a ribozyme, an anti-sense ribonucleic acid and an external guide sequence.

Preferably, the vector is a viral vector, more preferably a retroviral vector.

Preferably the detectable marker is a selectable marker, more preferably an enzyme capable of converting a prodrug into a cytotoxic compound. In a particularly preferred embodiment, the enzyme is thymidine kinase and the prodrug is gancyclovir.

The present invention further provides a plurality of viral particles, each viral particle comprising a first nucleotide sequence and/or a second nucleotide sequence as defined above.

The present invention also provides a method for producing plurality of viral particles as defined above which method comprises introducing into a producer cell (i) a plurality of first nucleotide sequences as defined above and (ii) a second nucleotide sequence as defined above. A plurality of viral particles produced by this method is also provided.

In a further aspect, the present invention provides a method for selecting from a plurality of first nucleotide sequences as defined above, a first nucleotide sequence encoding a gene product capable of binding to and effecting the cleavage, directly or indirectly, of a third nucleotide sequence, or a transcription product thereof; which method comprises:
(i) introducing one or more first nucleotide sequences into a host cell comprising a second nucleotide sequence as defined above; and
(ii) selecting the host cell if the detectable marker is not expressed in an active form in the host cell; and optionally,
(iii) isolating the first nucleotide sequence and determining its nucleotide sequence.

Preferably, in step (ii), the detectable marker is a selectable marker and the host cell is contacted with a compound such as a prodrug which is cytotoxic in the presence of the selectable marker. More preferably, the selectable marker is thymidine kinase and the prodrug is gancyclovir.

In a preferred embodiment, each first nucleotide sequence in the plurality of first nucleotide sequences is located downstream of a fourth nucleotide sequence encoding an inactive variant of the detectable marker such that any first nucleotide sequence(s) encoding a gene product capable of binding to and effecting the cleavage of a coding region encoding the detectable marker is removed.

In an especially preferred aspect of the invention, the plurality of first nucleotide sequences are present in a plurality of viral vectors, said viral vectors comprising a fourth nucleotide sequence encoding an inactive variant of the detectable marker, and as a preliminary step, the plurality of vectors are introduced into one or more producer cells and any resulting infectious viral particles used in step (i) to introduce the first nucleotides sequences into producer cells comprising a second nucleotide sequence.

The present invention provides in another aspect, a first nucleotide sequence obtained by the above method of the invention. Such a first nucleotide sequence may be used in therapy.

In a further aspect, the present invention provides a method for identifying an open site on a mRNA molecule, using a selection system according to the first aspect of the invention.

### Detailed Description of the Invention

### A. Components of selection system

### (i) Plurality of first nucleotide sequences encoding inhibitory RNA molecules

The first component of the selection system of the present invention is a plurality of nucleotide sequences which encode inhibitory RNA molecules. An inhibitory RNA molecule is defined as a ribonucleic acid which is capable of inhibiting transcription and/or translation of a nucleic acid sequence by binding to the sequence. Generally, an inhibitory RNA molecule is capable of binding to and effecting the cleavage, directly or indirectly, of a nucleotide sequence, or its transcription product. Examples include ribozymes (cleave directly), external guide sequences (EGSs) and anti-sense or sense RNA (cause cleavage by other factors).

The plurality of first nucleotide sequences encoding inhibitory RNA molecules will be substantially heterogeneous to create a diversity of sequences for the selection process. Thus, at least the part of the inhibitory molecule required for binding to a target nucleotide sequence will vary. Preferably, sequences required for catalytic activity, structural integrity and interactions with cellular components that degrade the target nucleotide sequence are not varied. However it may be convenient to produce the library by mutagenesis techniques which make randomly alterations throughout the inhibitory RNA molecule sequence. In the case of antisense and sense RNA constructs, this is in any case generally desirable. With regard to ribozymes and EGS sequences, it is preferred to generate the library by the use of randomly synthesised oligonucleotides which are then ligated into ribozyme or EGS constructs in the appropriate position. This results in a library of ribozymes/EGSs constructs where the region required for specificity varies whilst the other functional domains typically are constant.

By way of an example, a random ribozyme library can be generated by constructing oligonucleotides that contain the conserved enzymatic helix of the hammerhead ribozyme flanked with random nucleotides. The flanking sequences provide the recognition regions for the ribozyme. The length of each of the flanking sequences can vary although 8 nucleotides is a suitable length. This gives a complexity of approximately 16⁴ (65536) different molecules.

Suitable first nucleotide sequences for use according to the present invention encode gene products that result in the cleavage and/or enzymatic degradation of a target nucleotide sequence, which will generally be a ribonucleotide. As particular examples, ribozymes, external guide sequences and antisense sequences may be mentioned.

Ribozymes are RNA enzymes which cleave RNA at specific sites. Ribozymes can be engineered so as to be specific for any chosen sequence containing a ribozyme cleavage site. Thus, ribozymes can be engineered which have chosen recognition sites in transcribed viral sequences. By way of an example, ribozymes encoded by the first nucleotide sequence recognise and cleave essential elements of viral genomes required for the production of viral particles, such as packaging components and the mRNA genome. Thus, for retroviral genomes, such essential elements include the *gag, pol* and *env* gene products, and the viral mRNA genome. A suitable ribozyme capable of recognising at least one of the gag, pol and env gene sequences, or more typically, the RNA sequences transcribed from these genes, is able to bind to and cleave such a sequence. This will reduce or prevent production of the gal, pol or env protein as appropriate as well as the mRNA genome and thus reduce or prevent the production of retroviral particles.

Ribozymes come in several forms, including hammerhead, hairpin and hepatitis delta antigenomic ribozymes. Preferred for use herein are hammerhead ribozymes, in part because of their relatively small size, because the sequence requirements for their target cleavage site are minimal and because they have been well characterised. The ribozymes most commonly used in research at present are hammerhead and hairpin ribozymes.

Each individual ribozyme has a motif which recognises and binds to a recognition site in the target RNA. This motif takes the form of one or more "binding arms", generally two binding arms. The binding arms in hammerhead ribozymes are the flanking sequences Helix I and Helix III, which flank Helix II. These can be of variable length, usually between 6 to 10 nucleotides each, but can be shorter or longer. The length of the flanking sequences can affect the rate of cleavage. For example, it has been found that reducing the total number of nucleotides in the flanking sequences from 20 to 12 can increase the turnover rate of the ribozyme cleaving a HIV sequence, by 10-fold. A catalytic motif in the ribozyme Helix II in hammerhead ribozymes cleaves the target RNA at a site which is referred to as the cleavage site. Whether or not a ribozyme will cleave any given RNA is determined by the presence or absence of a recognition site for the ribozyme containing an appropriate cleavage site.

Each type of ribozyme recognises its own cleavage site. The hammerhead ribozyme cleavage site has the nucleotide base triplet GUX directly upstream where G is guanine, U is uracil and X is any nucleotide base. Hairpin ribozymes have a cleavage site of BCUGNYR, where B is any nucleotide base other than adenine, N is any nucleotide, Y is cytosine or thymine and R is guanine or adenine. Cleavage by hairpin ribozymes takes places between the G and the N in the cleavage site.

Antisense technology is well known in the art. One mechanism by which antisense sequences are believed to function is the recruitment of the cellular protein RNAseH to the target sequence/antisense construct heteroduplex which results in cleavage and degradation of the heteroduplex. Thus the antisense construct, by contrast to ribozymes, can be said to lead indirectly to cleavage/degradation of the target sequence. Thus according to the present invention, a first nucleotide sequence may encode an antisense RNA that binds to either a gene encoding an essential/packaging component or the RNA transcribed from said gene such that expression of the gene is inhibited, for example as a result of RNAseH degradation of a resulting heteroduplex. It is not necessary for the antisense construct to encode the entire complementary sequence of the gene encoding an essential/packaging component - a portion may suffice. The skilled person will easily be able to determine how to design a suitable antisense construct.

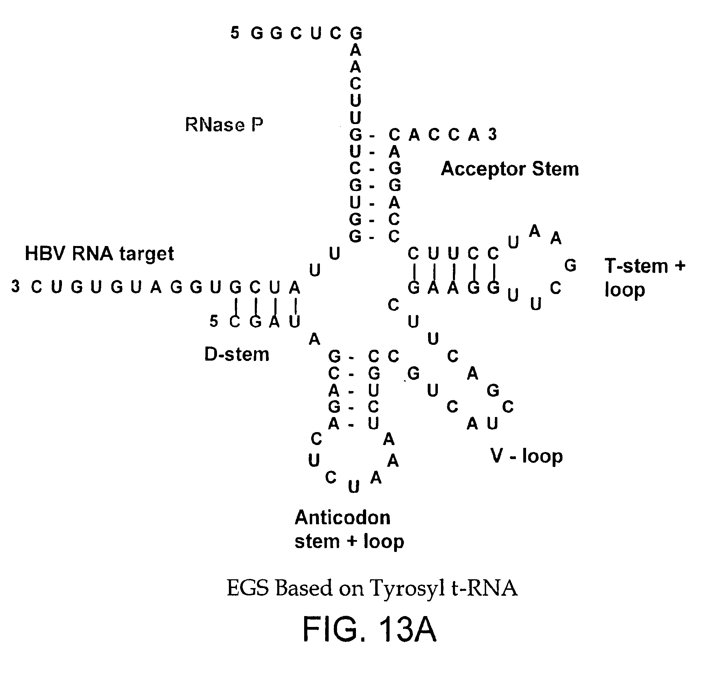

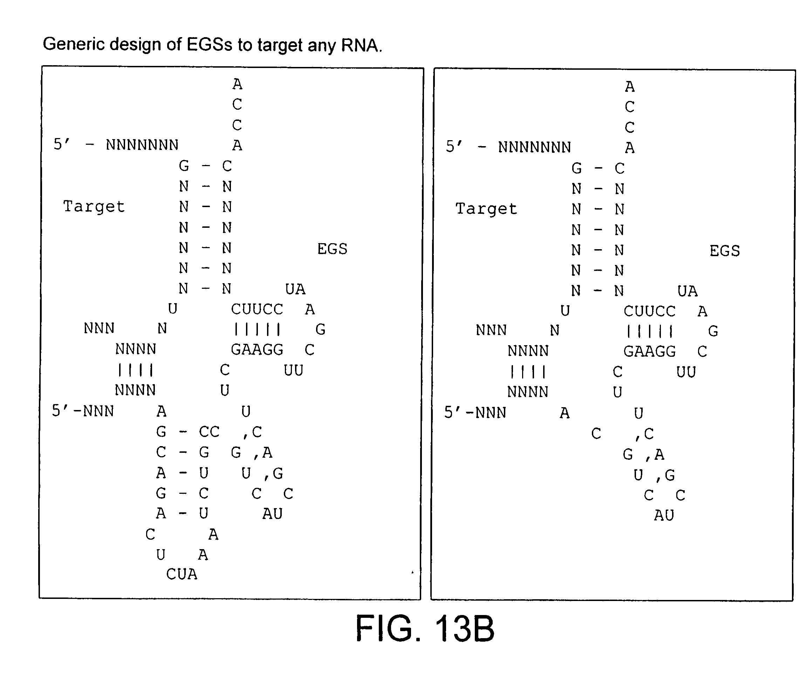

External guide sequences (EGSs) are RNA sequences that bind to a complementary target sequence to form a loop in the target RNA sequence, the overall structure being a substrate for RNaseP-mediated cleavage of the target RNA sequence. The structure that forms when the EGS anneals to the target RNA is very similar to that found in a tRNA precursor. The the natural activity of RNaseP can be directed to cleave a target RNA by designing a suitable EGS. The general rules for EGS design are as follows, with reference to the generic EGSs shown in Figure 13:

### Rules for EGS design in mammalian cells (see Figure 13)

Target sequence - All tRNA precursor molecules have a G immediately 3' of the RNaseP cleavage site (i.e. the G forms a base pair with the C at the top of the acceptor stem prior to the ACCA sequence). In addition a U is found 8 nucleotides downstream in all tRNAs. (i.e. G at position 1, U at position 8). A pyrimidine may be preferred 5' of the cut site. No other specific target sequences are generally required.

EGS sequence - A 7 nucleotide 'acceptor stem' analogue is optimal (5' hybridising arm). A 4 nucleotide 'D-stem' analogue is preferred (3' hybridising arm). Variation in this length may alter the reaction kinetics. This will be specific to each target site. A consensus 'T-stem and loop' analogue is essential. Minimal 5' and 3' non-pairing sequences are preferred to reduce the potential for undesired folding of the EGS RNA.

Deletion of the 'anti-codon stem and loop' analogue may be beneficial. Deletion of the variable loop can also be tolerated *in vitro* but an optimal replacement loop for the deletion of both has not been defined *in vivo.* Further guidance may be obtained by reference to, for example, Werner *et al.* (1997); Werner *et al*. (1998); Ma *et al.* (1998) and Kawa *et al.* (1998).

The nucleotide sequences will typically be part of a vector construct which will thus comprise a first nucleotide sequence encoding an inhibitory RNA molecule operably linked to a regulatory control sequence which permits expression of the inhibitory RNA molecule in the host cell. Further details of vector constructs, including viral vector constructs, are given below in section B.

### ii. Second nucleotide sequence comprising reporter constructs

The second nucleotide sequence in the system essential functions as a reporter construct. Thus the second nucleotide sequence comprises a coding region encoding a detectable marker. A detectable marker is any gene product which expression can be detected. Examples include lacZ, green fluorescent protein and selectable markers. Preferably the detectable marker is sortable using fluorescence activated cell sorting (FACS).

Selectable markers are typically gene products whose expression, or lack of, affects cell viability. Thus examples may include antibiotic resistance genes such as neomycin. Particularly preferred examples include gene products which are toxic to cells, either directly or via the conversion of a prodrug into a toxic compound. An example of the latter is the enzyme thymidine kinase which converts compounds such as gancyclovir into cytotoxic compounds. Thus is it preferred that the second nucleotide sequence encodes a gene product that is capable, when expressed, of reducing cell viability, including by causing cell death, either directly or in the presence of an exogenously added compound.

An important feature of the second nucleotide construct is that in addition to the coding region, at least one third nucleotide sequence is present either upstream or downstream of the coding sequence, which third nucleotide sequence it is desired to test the library of inhibitory RNA molecules against. For example, the third nucleotide sequence may be a component of a virus, such as a component of a retrovirus, such as a sequence encoding all or part of a gag.pol sequence. The presence of the third nucleotide sequence either upstream or downsteam of the detectable marker coding sequence means that in the event that one of the inhibitory RNA molecules encoded by the first nucleotide sequence binds to the third nucleotide sequence, or its transcription product, the resulting interaction prevents transcription of the second nucleotide sequence and/or causes a decrease in the stability of the mRNA produced from the second nucleotide sequence such that the expression of the detectable marker is reduced or inhibited. In the case of thymidine kinase, inhibition of TK expression removes the susceptibility of the cells to gancyclovir. Consequently, any surviving cells do not express sufficient TK to cause cell death in the presence of gancyclovir. When TK/gancyclovir are used, it is preferred to also administer dieldrin to minimise the bystander effect due to the cytotoxic compounds passing into neighbouring cells via gap junctions. Similar considerations apply to other cytotoxic compounds that have a bystander effect.

The third nucleotide sequence is therefore positioned relative to the coding sequence such that it is between the coding sequence and sequences required for mRNA stability and/or translation. For example, the third nucleotide sequence may be placed such that in the transcribed mRNA it is just downstream of the 5' cap or just upstream of the 3' polyA tail. An inhibitory RNA molecule which binds to and causes cleavage of the 5'cap and/or the polyA tail will enhance degradation of the remainder of the mRNA. Thus, the third nucleotide sequence will typically be present within the 5' and/or 3' untranslated regions (UTRs) of the mRNA encoding the detectable marker.

The third nucleotide sequence may be any nucleotide sequence for which it is desired to identify inhibitory RNA molecules which function efficiently against it. Thus, it may encode all or part of a gene product whose expression it is desired to inhibit or reduce. Such gene products may include mammalian cell gene products whose expression is associated with disease. Alternatively, the third nucleotide sequence may encode a component of a pathogenic organism, particularly a viral pathogen such as a retroviral pathogen, including HIV. Such components may, for example include gag.pol or env or accessory factors.

As for the first nucleotide sequence, the second nucleotide sequence comprising one or more third nucleotide sequences, is typically part of a nucleic acid vector as discussed in section B.

### B. Nucleic acid vectors

The first and second nucleotide sequences are typically present in nucleic acid vectors, which may be the same or different nucleic acid vectors, operably linked to a regulatory control sequence which permits expression of the first and second nucleotide sequences in a host cell.

The term "operably linked" means that the components described are in a relationship permitting them to function in their intended manner. A regulatory sequence "operably linked" to a coding sequence is ligated in such a way that expression of the coding sequence is achieved under condition compatible with the control sequences.

The control sequences may be modified, for example by the addition of further transcriptional regulatory elements to make the level of transcription directed by the control sequences more responsive to transcriptional modulators.

Vectors of the invention may be transformed or transfected into a suitable host cell as described below to provide for expression of the inhibitory RNA molecules. Vectors will be chosen that are compatible with the host cell used.

Control sequences operably linked to sequences encoding the inhibitory RNA molecules include promoters/enhancers and other expression regulation signals. These control sequences may be selected to be compatible with the host cell for which the expression vector is designed to be used in. The term promoter is well-known in the art and encompasses nucleic acid regions ranging in size and complexity from minimal promoters to promoters including upstream elements and enhancers.

The promoter is typically selected from promoters which are functional in mammalian cells, although promoters functional in other eukaryotic cells, such as insect cells, may be used. The promoter is typically derived from promoter sequences of viral or eukaryotic genes. For example, it may be a promoter derived from the genome of a cell in which expression is to occur. Viral promoters may also be used, for example the Moloney murine leukaemia virus long terminal repeat (MMLV LTR) promoter, the rous sarcoma virus (RSV) LTR promoter or the human cytomegalovirus (CMV) IE promoter.

The vector used in the method of the present invention may be may be non-viral, for example, plasmids, chromosomes or artificial chromosomes. Typical transfection methods include electroporation, DNA biolistics, lipid-mediated transfection, compacted DNA-mediated transfection, liposomes, immunoliposomes, lipofectin, cationic agent-mediated, cationic facial amphiphiles (CFAs) (Nature Biotechnology 1996 14; 556), and combinations thereof.

Alternatively, the vector may be a viral vector. Viral vectors include but are not limited to adenovirus vector, an adeno-associated viral (AAV) vector, a herpes viral vector, retroviral vector, lentiviral vector, baculoviral vector. The term "viral vector" refers to a nucleotide construct comprising a viral genome capable of being transcribed in a host cell, which genome comprises sufficient viral genetic information to allow packaging of the viral RNA genome, in the presence of packaging components, into a viral particle capable of infecting a target cell. Infection of the target cell includes reverse transcription and integration into the target cell genome, where appropriate for particular viruses. The viral vector in use typically carries heterologous coding sequences (nucleotides of interest) which are to be delivered by the vector to the target cell, for example a first nucleotide sequence encoding an inhibitory ribozyme. A viral vector is incapable of independent replication to produce infectious viral particles within the final target cell.

The term "viral vector system" is intended to mean a kit of parts which can be used when combined with other necessary components for viral particle production to produce viral particles in host cells. For example, the plurality of first nucleotide sequences may typically be present in a plasmid vector constructs suitable for cloning the first nucleotide sequences into a viral genome vector construct. When combined in a kit with a second nucleotide sequence (the reporter construct), which may be present in a separate plasmid vector construct, the resulting combination of the plurality of plasmids containing the first nucleotide sequence and plasmid containing the second nucleotide sequence comprises the essential elements of the invention. Such a kit may then be used by the skilled person in the production of suitable viral vector genome constructs which when transfected into a host cell together with the plasmids and optionally nucleic acid constructs encoding other components required for viral assembly, will lead to the production of infectious viral particles.

The second nucleotide sequence may also conveniently be present as part of the same vector construct as the first nucleotide construct. In this way, co-expression of the first nucleotide sequence and the reporter construct (such as the TK gene) in any individual cell is ensured. Similarly, viral vectors may also comprise both the first and second nucleotide sequences.

Alternatively, the second nucleotide sequence may be stably present within a packaging cell line that is included in the kit.

The kit may include the other components needed to produce viral particles, such as host cells and other plasmids encoding essential viral polypeptides required for viral assembly. By way of example, the kit may contain a plurality of plasmids containing a first nucleotide sequence encoding an anti-HIV ribozyme and a second nucleotide sequence encoding TK operably linked to a third nucleotide sequence placed within the 5' and/or 3' UTR. Optional components would then be (a) an HIV viral genome construct with suitable restriction enzyme recognition sites for cloning the first and second nucleotide sequences into the viral genome; (b) a plasmid encoding a VSV-G env protein and (c) a plasmid encoding gag.pol. Alternatively, nucleotide sequence encoding viral polypeptides required for assembly of viral particles may be provided in the kit as packaging cell lines comprising the nucleotide sequences, for example a VSV-G expressing cell line.

Viral vectors are typically retroviral vectors, in particular lentiviral vectors such as HIV vectors. The retroviral vector of the present invention may be derived from or may be derivable from any suitable retrovirus. A large number of different retroviruses have been identified. Examples include: murine leukemia virus (MLV), human immunodeficiency virus (HIV), simian immunodeficiency virus, human T-cell leukemia virus (HTLV), equine infectious anaemia virus (EIAV), mouse mammary tumour virus (MMTV), Rous sarcoma virus (RSV), Fujinami sarcoma virus (FuSV), Moloney murine leukemia virus (Mo-MLV). FBR murine osteosarcoma virus (FBR MSV), Moloney murine sarcoma virus (Mo-MSV), Abelson murine leukemia virus (A-MLV). Avian myelocytomatosis virus-29 (MC29), and Avian eryhroblastosis virus (AEV). A detailed list of retroviruses may be found in Coffin *et al*., 1997. "Retroviruses". Cold Spring Harbour Laboratory Press Eds: JM Coffin. SM Hughes, HE Varmus pp 758-763.

Details on the genomic structure of some retroviruses may be found in the art. By way of example, details on HIV and Mo-MLV may be found from the NCBI Genbank (Genome Accession Nos. AF033819 and AF033811, respectively).

The lentivirus group can be split even further into "primate" and "non-primate". Examples of primate lentiviruses include human immunodeficiency virus (HIV), the causative agent of human auto-immunodeficiency syndrome (AIDS), and simian immunodeficiency virus (SIV). The non-primate lentiviral group includes the prototype "slow virus" visna/maedi virus (VMV), as well as the related caprine arthritis-encephalitis virus (CAEV), equine infectious anaemia virus (EIAV) and the more recently described feline immunodeficiency virus (FIV) and bovine immunodeficiency virus (BIV).

The basic structure of a retrovirus genome is a 5' LTR and a 3' LTR, between or within which are located a packaging signal to enable the genome to be packaged, a primer binding site, integration sites to enable integration into a host cell genome and *gag*, *pol* and *env* genes encoding the packaging components - these are polypeptides required for the assembly of viral particles. More complex retroviruses have additional features, such as *rev* and RRE sequences in HIV, which enable the efficient export of RNA transcripts of the integrated provirus from the nucleus to the cytoplasm of an infected target cell.

In the provirus, these genes are flanked at both ends by regions called long terminal repeats (LTRs). The LTRs are responsible for proviral integration, and transcription. LTRs also serve as enhancer-promoter sequences and can control the expression of the viral genes. Encapsidation of the retroviral RNAs occurs by virtue of a *psi* sequence located at the 5' end of the viral genome.

The LTRs themselves are identical sequences that can be divided into three elements, which are called U3. R and U5. U3 is derived from the sequence unique to the 3' end of the RNA. R is derived from a sequence repeated at both ends of the RNA and U5 is derived from the sequence unique to the 5' end of the RNA. The sizes of the three elements can vary considerably among different retroviruses.

In a defective retroviral vector genome *gag*, *pol* and *env* may be absent or not functional. The R regions at both ends of the RNA are repeated sequences. U5 and U3 represent unique sequences at the 5' and 3' ends of the RNA genome respectively.

In a typical retroviral vector for use in gene therapy and/or other techniques of molecular biology, at least part of one or more of the *gag, pol* and *env* protein coding regions essential for replication may be removed from the virus. This makes the retroviral vector replication-defective. The removed portions may even be replaced by a nucleotide sequence of interest (NOI), such as a first nucleotide, to generate a virus capable of integrating its genome into a host genome but wherein the modified viral genome is unable to propagate itself due to a lack of structural proteins. When integrated in the host genome, expression of the NOI occurs - resulting in, for example, a therapeutic and/or a diagnostic effect. Thus, the transfer of an NOI into a site of interest is typically achieved by: integrating the NOI into the recombinant viral vector; packaging the modified viral vector into a virion coat; and allowing transduction of a site of interest - such as a targeted cell or a targeted cell population.

A minimal retroviral genome for use in the present invention will therefore comprise (5') R - U5 - a first nucleotide sequence and/or a second nucleotide sequence - U3-R (3'). However, the plasmid vector used to produce the retroviral genome within a host cell/packaging cell will also include transcriptional regulatory control sequences operably linked to the retroviral genome to direct transcription of the genome in a host cell/packaging cell. These regulatory sequences may be the natural sequences associated with the transcribed retroviral sequence, i.e. the 5' U3 region, or they may be a heterologous promoter such as another viral promoter, for example the CMV promoter.

Some retroviral genomes require additional sequences for efficient virus production. For example, in the case of HIV, *rev* and RRE sequence are preferably included. However the requirement for *rev* and RRE can be reduced or eliminated by codon optimisation.

Once the retroviral vector genome is integrated into the genome of its target cell as proviral DNA. the ribozyme sequences need to be expressed. In a retrovirus, the promoter is located in the 5' LTR U3 region of the provirus. In retroviral vectors, the promoter driving expression of a heterologous gene may be the native retroviral promoter in the 5' U3 region, or an alternative promoter engineered into the vector. The alternative promoter may physically replace the 5' U3 promoter native to the retrovirus, or it may be incorporated at a different place within the vector genome such as between the LTRs.

Replication-defective retroviral vectors are typically propagated, for example to prepare suitable titres of the retroviral vector for subsequent transduction, by using a combination of a packaging or helper cell line and the recombinant vector. That is to say, that the three packaging proteins can be provided *in trans.*

A "packaging cell line" contains one or more of the retroviral *gag, pol* and *env* genes. The packaging cell line produces the proteins required for packaging retroviral DNA but it cannot bring about encapsidation due to the lack of a *psi* region. However, when a recombinant vector carrying an NOI and a *psi* region is introduced into the packaging cell line, the helper proteins can package the *psi*-positive recombinant vector to produce the recombinant virus stock. This virus stock can be used to transduce cells to introduce the NOI into the genome of the target cells. It is preferred to use a *psi* packaging signal, called *psi* plus, that contains additional sequences spanning from upstream of the splice donor to downstream of the *gag* start codon (Bender *et al*., 1987) since this has been shown to increase viral titres.

The recombinant virus whose genome lacks all genes required to make viral proteins can tranduce only once and cannot propagate. These viral vectors which are only capable of a single round of transduction of target cells are known as replication defective vectors. Hence, the NOI is introduced into the host/target cell genome without the generation of potentially harmful retrovirus. A summary of the available packaging lines is presented in Coffin *et al*., 1997 (*ibid*).

Retroviral packaging cell lines in which the *gag*, *pol* and *env* viral coding regions are carried on separate expression plasmids that are independently transfected into a packaging cell line are preferably used. This strategy, sometimes referred to as the three plasmid transfection method (Soneoka *et al*., 1995), reduces the potential for production of a replication-competent virus since three recombinant events are required for wild type viral production. As recombination is greatly facilitated by homology, reducing or eliminating homology between the genomes of the vector and the helper can also be used to reduce the problem of replication-competent helper virus production.

An alternative to stably transfected packaging cell lines is to use transiently transfected cell lines. Transient transfections may advantageously be used to measure levels of vector production when vectors are being developed. In this regard, transient transfection avoids the longer time required to generate stable vector-producing cell lines and may also be used if the vector or retroviral packaging components are toxic to cells. Components typically used to generate retroviral vectors include a plasmid encoding the gag/pol proteins, a plasmid encoding the env protein and a plasmid containing an NOI. Vector production involves transient transfection of one or more of these components into cells containing the other required components. If the vector encodes toxic genes or genes that interfere with the replication of the host cell, such as inhibitors of the cell cycle or genes that induce apotosis, it may be difficult to generate stable vector-producing cell lines, but transient transfection can be used to produce the vector before the cells die. Also, cell lines have been developed using transient transfection that produce vector titre levels that are comparable to the levels obtained from stable vector-producing cell lines.

Producer cells/packaging cells can be of any suitable cell type. Most commonly, mammalian producer cells are used but other cells, such as insect cells are not excluded. Clearly, the producer cells will need to be capable of efficiently translating the env and gag, pol mRNA. Many suitable producer/packaging cell lines are known in the art. The skilled person is also capable of making suitable packaging cell lines by, for example stably introducing a nucleotide construct encoding a packaging component into a cell line.

Where the first nucleotide sequence within the retroviral genome encodes an inhibitory RNA molecule which is being tested for it ability to effect the cleavage of *gag, pol* and/or *env* RNA transcripts, the nucleotide sequences present in the packaging cell line, either integrated or carried on plasmids, or in the transiently transfected producer cell line, which encode gag, pol and or env proteins may be modified so as to reduce or prevent binding of the inhibitory RNA molecule(s). In this way, the inhibitory RNA molecule(s) will not prevent expression of viral polypeptides in packaging cell lines that are essential for packaging of viral particles. Clearly, given the random nature of the library, it is still possible that some sequences within the library will cleave modified packaging components but by having the third nucleotide sequence and the sequence encoding the corresponding packaging component different, an inhibitory molecule which affect the modified packaging component is less likely also simultaneously to affect the third nucleotide sequence and therefore the cell will in many cases produce the detectable marker and be eliminated from the screen.

The term "viral polypeptide essential for packaging of viral particles" means a polypeptide normally encoded by the viral genome to be packaged into viral particles, in the absence of which the viral genome cannot be packaged. For example, in the context of retroviruses such polypeptides would include gag, pol and env. The terms "packaging component" and "essential component" are also included within this definition.

By way of example, in the case of ribozymes, resistance is typically by virtue of alterations in the sequences which eliminate the ribozyme recognition sites. At the same time, the amino acid coding sequence for the essential/packaging components is retained so that the viral components encoded by the sequences remain the same, or at least sufficiently similar that the function of the essential/packaging components is not compromised. Again, given the often random nature of the plurality of first nucleotide sequences, extensive modification of the nucleotide sequence encoding the packing component may be required.

In the case of antisense sequences, the third nucleotide sequence differs from the nucleotide sequence encoding the viral packaging component to the extent that although an antisense sequence can bind to the third nucleotide sequence, or transcript thereof, the antisense sequence can not bind effectively to the nucleotide sequence encoding the required packaging component or RNA transcribed from therefrom. The differences between the third nucleotide sequences and the nucleotide sequences encoding a required packaging component will typically be conservative changes, although a small number of amino acid changes may be tolerated provided that, as described above, the function of the essential/packaging components is not significantly impaired.

More generally, a suitable approach may be to alter the nucleotide sequence of the required packaging component so that the nucleotide homology over the region corresponding to the third nucleotide sequence is less than 95%, preferably less than 90, 80 or 70% whilst the amino acid homology is preferably at least 95%.

Preferably, in addition to eliminating the inhibitory RNA recognition sites, the alterations to the coding sequences for the viral components improve the sequences for codon usage in the mammalian cells or other cells which are to act as the producer cells for retroviral vector particle production. This improvement in codon usage is referred to as "codon optimisation". Many viruses, including HIV and other lentiviruses, use a large number of rare codons and by changing these to correspond to commonly used mammalian codons, increased expression of the packaging components in mammalian producer cells can be achieved. Codon usage tables are known in the art for mammalian cells, as well as for a variety of other organisms.

Thus preferably, the sequences encoding the packaging components are codon optimised. More preferably, the sequences are codon optimised in their entirety. Following codon optimisation, it is found that there are numerous sites in the wild type *gag, pol* and *env* sequences which can serve as inhibitory RNA recognition sites and which are no longer present in the sequences encoding the packaging components.

An additional advantage of codon optimising HIV packaging components is that this can increase gene expression. In particular, it can render *gag, pol* expression Rev independent so that *rev* and RRE need not be included in the genome (Haas *et al*., 1996). Rev-independent vectors are therefore possible. This in turn enables the use of anti-*rev* or RRE factors in the retroviral vector.

It is highly desirable to use high-titre virus preparations in both experimental and practical applications. Techniques for increasing viral titre include using a *psi* plus packaging signal as discussed above and concentration of viral stocks. In addition, the use of different envelope proteins, such as the G protein from vesicular-stomatitis virus has improved titres following concentration to 10⁹ per ml. However, typically the envelope protein will be chosen such that the viral particle will preferentially infect cells that are infected with the virus which it desired to treat. For example where an HIV vector is being used to treat HIV infection, the env protein used will be the HIV env protein.

### C. Selection methods

The selection method of the invention involves introducing one or more first nucleotide sequences into a host cell in the presence of a second nucleotide sequence. Methods for introducing nucleic acids into host cells are well known in the art, for example transfection. Generally, the nucleotide sequences are part of a vector. The first and second nucleotides sequences may be part of the same vector or present on separate nucleic acid molecules. The second nucleotide sequence may even be stably introduced into the genome of the host cell.

If an inhibitory RNA molecule encoded by a first nucleotide sequence inhibits or reduces expression of a detectable marker then it will be possible to distinguish cells containing such a molecule from cells that contain a first nucleotide sequence that does not lead to an inhibition in or reduction of expression of the detectable marker. Cells that contain the effective inhibitory RNA molecule can be used in, for example, a PCR reaction, to identify the sequence of the inhibitory RNA molecule and thus the sequence of the region of the target molecule.

If the detectable marker is FACS-sortable, then the cells which contain an effective inhibitory RNA molecule can be sorted from the remainder of the cells. If the selectable marker is an enzyme capable of converting a prodrug into a cytotoxic compound, those cells which contain an effective inhibitory RNA molecule will be selectable based on their insensitivity to the prodrug. More specifically, those cells which contain an effective inhibitory RNA molecule will be better able to survive selection with the prodrug.

In a preferred embodiment, the latter selection process is conveniently performed using a library of viral vectors comprising first nucleotide sequences. The viral vector library is transfected into producer cells comprising the second nucleotide sequence encoding, for example. TK. Cells which survive subsequent prodrug (for example, gancyclovir) selection will generally comprise at least one first nucleotide sequence encoding an effective inhibitory RNA molecule. A further optional stage is to harvest the viral particles from said cells and use the infectious viral particles to transduce further cells. An advantage of this extra step is that, typically, only first nucleotide sequences that do not cleave essential viral sequences/packaging components will be carried through successfully to this second stage. In addition, by making a dilution series of the viral supernatant obtained from the viable cells, it may be possible to produce cells comprising only one first inhibitory sequence which will simplify identification by PCR.

It should be noted that some inhibitory molecules may cause cell death for other reasons other than by failure to target a third nucleotide sequence. For example an inhibitory molecule may cause degradation of an mRNA essential for cell survival. In general, since cells containing such molecules will die, selection against these elements of a random library is not required.

"False positives" may also be obtained if the first nucleotide sequence is capable of binding to and effecting the cleavage of the coding region encoding the detectable marker. For example, if the selection process depends on the capacity of those cells which contain an effective inhibitory RNA molecule to survive selection with a prodrug, false positives may be obtained if the inhibitory molecule(s) directly target the mRNA encoding the enzyme which converts the prodrug into a cytotoxic compound. In an especially preferred embodiment, a selection step is included to eliminate vectors encoding inhibitory molecules that target sequences of the detectable marker used in the second nucleic acid.

This can be done, for example, by inserting the first nucleotide sequences into a construct comprising a non-functional nucleotide sequence encoding a detectable marker and lacking the third nucleotide sequence ("a fourth nucleotide sequence"). Preferably, the other vector sequences are identical to those of the vector comprising the second nucleotide sequence. Typically, the nucleotide sequence encoding the detectable marker has been modified by insertion or deletion to prevent expression of a functional detectable marker. The inactive detectable marker is thus the same sequence as detectable marker sequence used in the second nucleic acid, apart from the mutation which renders it non-functional.

The construct can then be used to generate a library viral vectors for the initial screening stage. The genomes encoding inhibitory molecules that (i) are capable of binding to and causing cleavage of the detectable marker; (ii) affect the viral genome in which they are contained; (iii) affect viral packaging; or (iv) kill cells, will not be packaged into viral particles. Thus a pool of viruses may be produced that comprise a plurality of first nucleotide sequences preselected so as not to be (i) capable of causing cleavage of the detectable marker; (ii) detrimental to cell survival, or (iii) capable of inhibiting viral production in the selection assay.

This pool of viruses can then be used to transduce cells comprising the second nucleotide sequence in a selection round as described above.

### D. Uses of optimised inhibitory RNA molecules

Optimised inhibitory RNA molecules, such as ribozymes, identified by the selection method of the invention, may be used to inhibit expression of their target nucleotide sequence or transcription product thereof in a target cell.

This may be used to ablate the expression of genes in cells in vitro or in vivo in order to investigate the effect of blocking gene expression, for example by conducting "knock-out" experiments. Such experiments are useful, for example, for target validation, screening or model building.

Alternatively, optimised inhibitory RNA molecules identified by the selection method of the invention may be used in therapeutic applications such as gene therapy and direct administration of RNA. In particular, optimised inhibitory RNA molecules which target nucleotide sequence encoding essential components of pathogens may be used to treat disease caused by said pathogens. Thus an optimised inhibitory RNA molecule that targets a component of HIV may be used to reduce or prevent an HIV infection or associated symptoms.

Thus nucleic acid vectors comprising nucleotide sequences encoding optimised inhibitory RNA molecules may be used to treat or prevent viral infections, preferably retroviral infections, in particular lentiviral, especially HIV, infections. Specifically, said nucleic acid vectors may be used to deliver inhibitory RNA molecules to a human or animal in need of treatment for a viral infection. Nucleic acid vectors include viral vectors and infectious viral particles obtained therefrom. Where the viral vector comprises a selected first nucleotide sequence which targets a component of a pathogenic virus, which component is also required for the generation of infectious viral particles comprising the selected first nucleotide sequence, the nucleotide sequence of the component in packaging/producer cells will typically be modified as described above. In particular, guidance is given in the reference examples with respect to the use of ribozymes and modified retroviral env and gag.pol sequences.

HIV-1 provides an ideal vector for the development of strategies to combat HIV-1 infection. This is because a genome with the open reading frames removed and packaged by trans-produced viral particles (hence non-pathogenic, but still immunogenic) can be used to carry anti-HIV molecules such as ribozymes, intra-bodies, intra-kines, RNA decoys and/or trans-dominant mutated proteins.

If the splice donor and splice acceptor sites are retained then up to 8 distinct anti-HIV factors could be generated by one viral construct. For example the Tat reading frame could be replaced with a ribozyme targeting Tat and the nef reading frame be replaced with a tat transdominant mutant.

Inhibitory RNA molecules such as ribozymes would be a key component of such a system.

Preferably the nucleic acid vectors/viral particles are combined with a pharmaceutically acceptable carrier or diluent to produce a pharmaceutical composition. Thus, the present invention also provides a pharmaceutical composition for treating an individual, wherein the composition comprises a therapeutically effective amount of the nucleic acid vector/viral particle of the present invention, together with a pharmaceutically acceptable carrier, diluent, excipient or adjuvant. The pharmaceutical composition may be for human or animal usage.

The choice of pharmaceutical carrier, excipient or diluent can be selected with regard to the intended route of administration and standard pharmaceutical practice. Suitable carriers and diluents include isotonic saline solutions, for example phosphate-buffered saline. The pharmaceutical compositions may comprise as - or in addition to - the carrier, excipient or diluent any suitable binder(s), lubricant(s), suspending agent(s), coating agent(s), solubilising agent(s), and other carrier agents that may aid or increase the viral entry into the target site (such as for example a lipid delivery system).

The pharmaceutical composition may be formulated for parenteral, intramuscular, intravenous, intracranial, subcutaneous, intraocular or transdermal administration.

Where appropriate, the pharmaceutical compositions can be administered by any one or more of: inhalation, in the form of a suppository or pessary, topically in the form of a lotion, solution, cream, ointment or dusting powder, by use of a skin patch, orally in the form of tablets containing excipients such as starch or lactose, or in capsules or ovules either alone or in admixture with excipients, or in the form of elixirs, solutions or suspensions containing flavouring or colouring agents, or they can be injected parenterally, for example intracavernosally, intravenously, intramuscularly or subcutaneously. For parenteral administration, the compositions may be best used in the form of a sterile aqueous solution which may contain other substances, for example enough salts or monosaccharides to make the solution isotonic with blood. For buccal or sublingual administration the compositions may be administered in the form of tablets or lozenges which can be formulated in a conventional manner.

The amount of virus administered is typically in the range of from 10³ to 10¹⁰ pfu, preferably from 10⁵ to 10⁸ pfu, more preferably from 10⁶ to 10⁷ pfu. When injected, typically 1-10 µl of virus in a pharmaceutically acceptable suitable carrier or diluent is administered.

When the polynucleotide/vector is administered as a naked nucleic acid, the amount of nucleic acid administered is typically in the range of from 1 µg to 10 mg, preferably from 100 µg to 1 mg.

Where a first nucleotide sequence selected by the method of the invention (or other therapeutic sequence) is under the control of an inducible regulatory sequence, it may only be necessary to induce gene expression for the duration of the treatment. Once the condition has been treated, the inducer is removed and expression of the nucleotide sequence is stopped. This will clearly have clinical advantages. Such a system may, for example, involve administering the antibiotic tetracycline, to activate gene expression via its effect on the tet repressor/VP 16 fusion protein.

### E. In vivo selection methods

In one aspect, the present invention provides a method for identifying an open site on a mRNA molecule.

The term "open" means a site which, when the mRNA is in its physiological confirmation, is accessible to inhibitory RNA molecules such as ribozymes, antisense RNA or EGSs.

Using the selection system of the present invention, the mRNA is encoded by the third nucleotide sequence. For example, if the third nucleotide sequence is a whole gene, the selection system can be used to identify one or more open sites on the corresponding mRNA molecule. Once an inhibitory RNA molecule (such as ribozymes, antisense RNA or EGSs) has been identified as capable of binding to the third nucleotide sequence using the selection system, the target site can be deduced using standard techniques. For example, if the inhibitory molecule is an antisense sequence, a sequence comparison between the "optimum" complementary sequence of the antisense sequence, and the mRNA sequence should reveal the site at which the antisense sequence bound to the mRNA.

The invention will now be further described by way of Examples, which are meant to serve to assist one of ordinary skill in the art in carrying out the invention and are not intended in any way to limit the scope of the invention. The Examples refer to the Figures. In the

Figures:
Figure 1. Prodrug dose response - Graph of percentage survival vs prodrug concentration for: A. *E. coli* Nitroreductase (NTR)/Metronidazole (MTZ) and B. HSV-1 Thymidine kinase (TK)/Gancyclovir (GCV).
Figure 2. Bystander Effect - Graph of percentage survival vs prodrug concentration for A. NTR/MTZ and B. TK/GCV
Figure 3 shows a bar chart of % survival for HeLa cell lines comprising CTKtatSN which have been transduced with pH4Z (tat-4Z), an anti-tat ribozyme with the catalytic activity removed (tat-RzM) or an anti-tat ribozyme (tat-Rz). The cells have been incubated with GCV or GCV and dieldrin.
Figure 4 shows a schematic representation of vector genomes.
Figure 5 shows schematically ribozymes inserted into four different HIV vectors;
Figure 6 shows schematically how to create a suitable 3' LTR by PCR;
Figure 7 shows the codon usage table for wild type HIV *gag,pol* of strain HXB2 (accession number: K03455).
Figure 8 shows the codon usage table of the codon optimised sequence designated gag,pol-SYNgp.
Figure 9 shows the codon usage table of the wild type HIV *env* called env-mn.
Figure 10 shows the codon usage table of the codon optimised sequence of HIV *env* designated SYNgp160mn.
Figure 11 shows three plasmid constructs for use in the invention.
Figure 12 shows the principle behind two systems for producing retroviral vector particles.
Figure 13A.B - design of external guide sequences.

The invention will now be further described in the Examples which follow, which are intended as an illustration only and do not limit the scope of the invention.

### EXAMPLES

The present inventors have developed a method which is based on the generation of a library of ribozymes each containing the catalytic domain of a hammerhead ribozyme. The procedure involves generating a virus that contains a genome comprising a nucleotide sequence that encodes a suicide gene (thymidine kinase (TK) is preferred). The target sequence is inserted downstream of this. Alternatively, or in addition, if an internal ribosome entry site (IRES) is inserted upstream of the TK sequence then the target sequence can be inserted upstream. Cells that are transduced with this vector will be sensitive to gancyclovir.

However, when the viral genome is transcribed the mRNA contains not only the TK gene but also the target sequence. This mRNA will contain the normal 5' cap and the 3' poly A tail. If the 5' cap or the poly A tail are removed by cleavage with a ribozyme the mRNA is unstable and is degraded. This prevents the translation of the TK gene, rendering cells that express an active ribozyme insensitive to gancyclovir.

### Example 1 - selection of prodrug system (selectable marker)

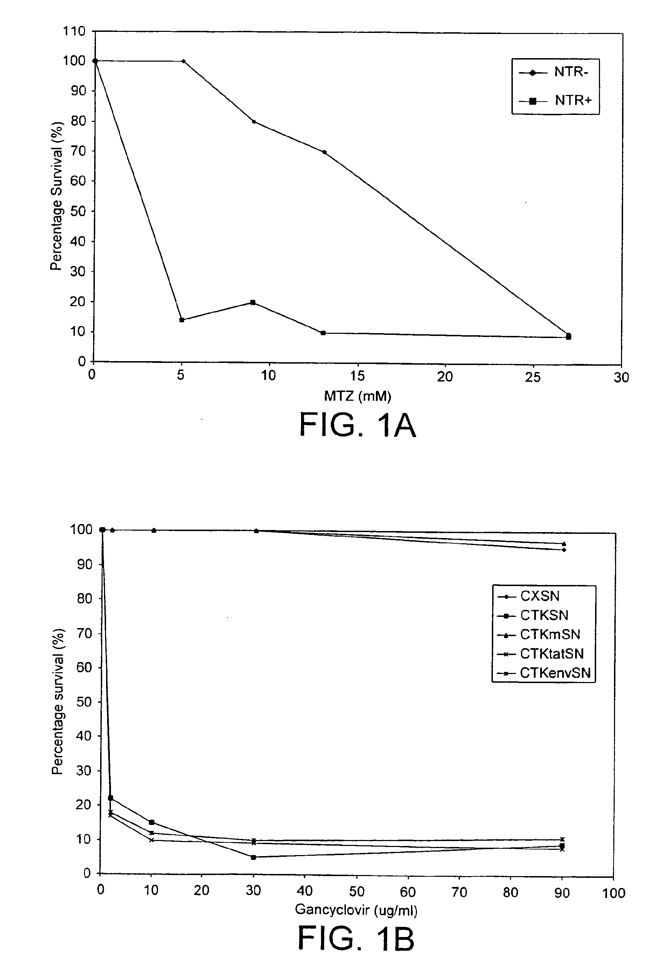

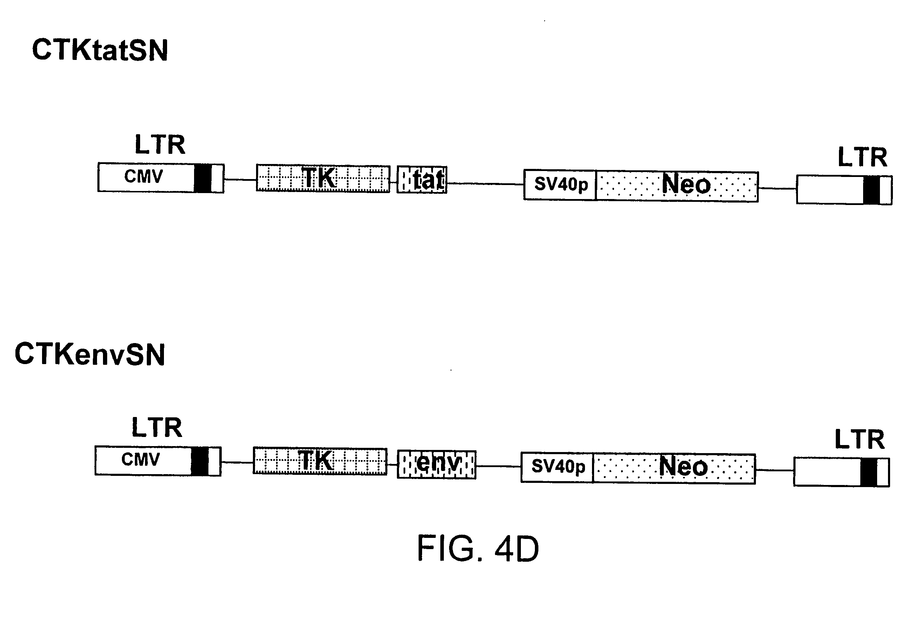

The second nucleotide used in the present invention encodes a detectable marker. We have chosen to exemplify the present invention using a enzyme-prodrug selection system. The dose response of two well-known enzyme-prodrug combinations was measured to select an effective prodrug system.
A. An *E. coli* Nitroreductase (NTR)/Metronidazole (MTZ) combination: HT1080 cells (NTR-) and HT1080 NTR positive cells (NTR+) were incubated with varying concentrations of MTZ and the percentage of cells which survived was measured (Figure 1A).
B. An HSV-1 Thymidine kinase (TK)/Gancyclovir (GCV) combination: HeLa cell lines transduced with various viral vectors were treated for 72 hours with GCV and the percentage of cells which survived was measured (see Figure 1B). CXSN = empty viral vector, CTKSN = TK positive virus, CTKmSN = TK mutant virus, CTKtatSN = vector containing the HIV-1 tat gene ORF downstream of an active TK gene (Figure 4D), CTKenvSN = vector containing the HIV-1 env gene ORF downstream of an active TK gene (Figure 4D). CXSN is made by removing the lac Z gene from pHIT111. C = CMV promoter in place of the 5' U3, X = cloning site, for the gene of interest, S = SV40 promoter and N = Neomycin resistance gene. Tk = thymidine kinase, TKm = thymidine kinase mutant.

These results indicate that better sensitivity can be obtained with a TK/GCV system. However, TK/GCV produces a bystander effect. Generally, the drug used should not have a bystander effect, i.e. cause the death of cells surrounding the cell that is expressing the enzyme that activates the prodrug. The bystander effect is due to the active form of the prodrug either crossing the plasma membrane and entering surrounding cells or entering other cells through gap junctions. In the case of GCV the bystander effect is caused by the drug crossing through gap junctions. Fortunately, these gap junctions can be blocked with the drug dieldrin and hence limit the bystander effect (Touraine *et al*., 1998).

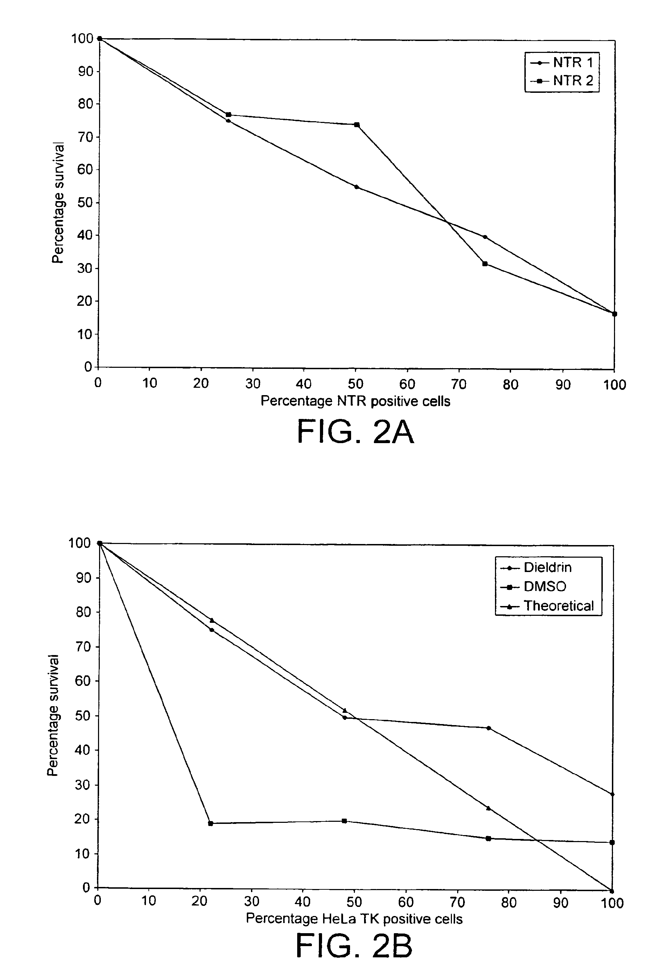

Figure 2 shows the bystander effect of the two enzyme/prodrug systems mentioned above. In Figure 2A, an HT1080 NTR positive stable cell line was co-cultured with HT1080 cells in the presence of 10 mM MTZ for 24 hours. In Figure 2B, a HeLa TK stable cell line was co-cultured with HeLa cell with GCV in the presence of absence of dieldrin (16 µg/ml) for 72 hours.

From this data it is clear that the TK/GCV bystander effect can be minimised using dieldrin.

Thus, we have chosen to use HSV-1 TK/GCV/dieldrin, in preference to NTR/metronidazole (MTZ) which does not have a bystander effect, because TK/GCV is more sensitive in distinguishing cells with or without the enzyme (Figure 1A, 1B). The amount of GCV used in the study is 3 µg/ml as determined from Figure 1.

### Example 2 - In vivo selection using hammerhead ribozymes

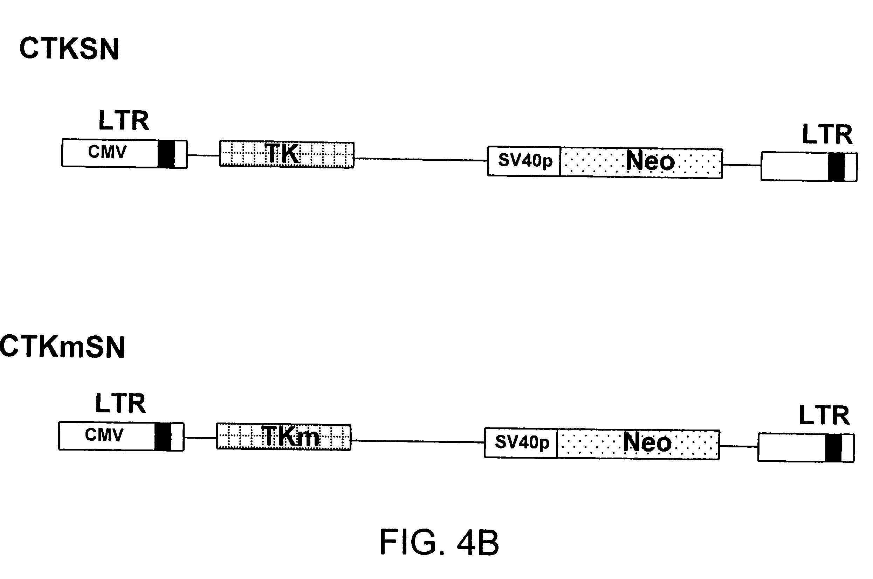

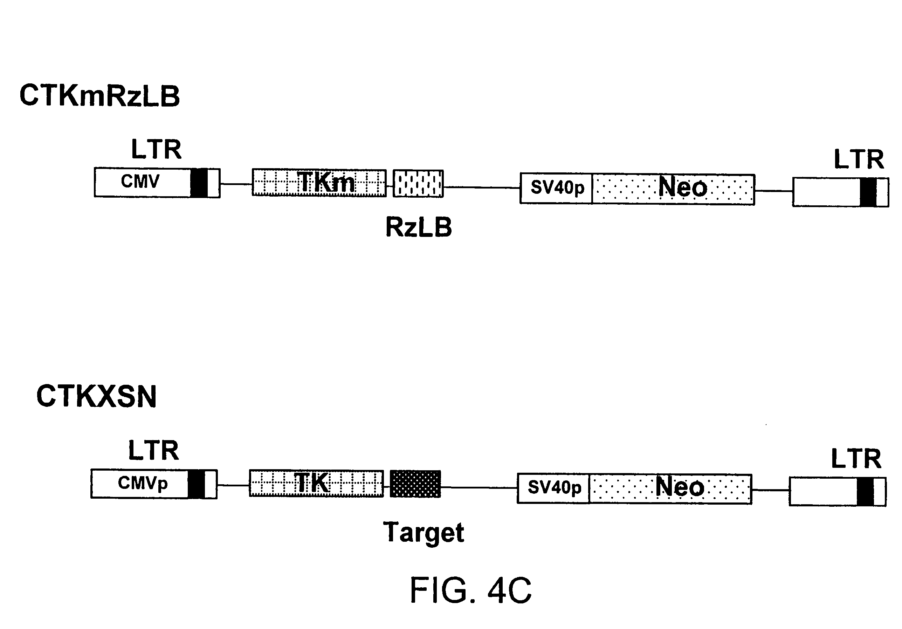

A library of hammerhead ribozymes is generated by random oligonucleotide synthesis. These are then inserted into CTKmSN (Figure 4B) downstream of the inactive TK coding sequence to give a plurality of enzymes (CTKmRzLB - Figure 4C).

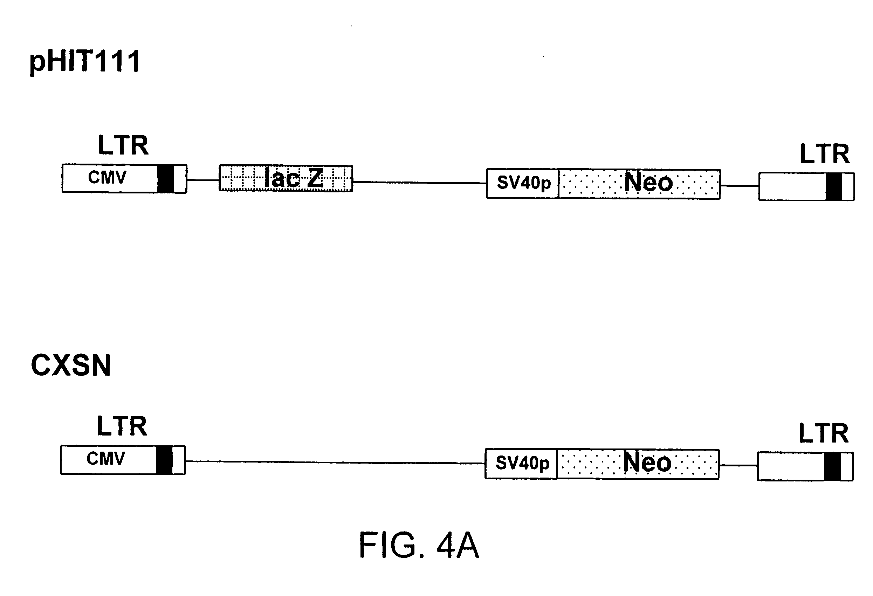

CTKmSN (Figure 4B) was constructed by removing the lacZ of pHIT111 (Soneoka *et al*., 1995) (Figure 4A) to give the intermediate construct CKSN (Figure 4A) and then inserting in its place an inactive TK coding sequence constructed by a frameshift mutation (cut and re-fill at BsP EI site). The sequence of human herpes virus 1 is provided in Wagner *et al*., 1981 (Genbank accession no. V00467).

Insertion of the library downstream of the inactive form of the enzyme is necessary to ensure that no ribozymes that cut the TK containing RNA (and therefore would lead to false positives in the selection) are contained in the vector library. Virus was generated by the three plasmid co-transfection system (Soneoka *et al*., 1995) and used to transduce HeLa cells. The activity of the mutant was assessed by measuring cell survival after exposure to GCV.

The target RNA sequences are inserted in the viral vector CTKSN (Figure 4B) downstream of the active form of the enzyme, to make CTKXSN where X = target sequence (Figure 4C). CTKSN is made by inserting the TK sequence in CXSN (Figure 4A). Following three plasmid co-tranfection the virus is used to transduce cells such as NIH3T3. HeLa or any other cells. HeLa cells were chosen in order to minimise bystander effects (HeLa cells have a low percentage of gap-junctional communication and the bystander effect of GCV is due to transmission of the monophosphate guanosine analog through gap junctions). Dieldrin was also used to minimise the bystander effect.

Stable cell lines are generated by neomycin selection (G418, 1 mg/ml for 10 days). These cell lines can now be transduced with the vector containing the ribozyme library (CTKmRxLB) and GCV selection will follow. Any ribozymes that cut the HIV sequence will prevent translation of TK and hence the cells will survive. All of the other cells will die due to retaining functional TK. PCR can then be used to establish the ribozyme sequence.

### Example 3 - In vivo testing using optimised ribozymes

The optimised ribosomes obtained according to Example 1 may be used in the treatment of diseases. In particular, a number of these ribozymes may be used in tandem allowing the targeting of a number of sites. In addition, in the treatment of HIV, an HIV vector can be used to deliver the ribozymes, as this will cause interference with the packaging of the wild type genome.

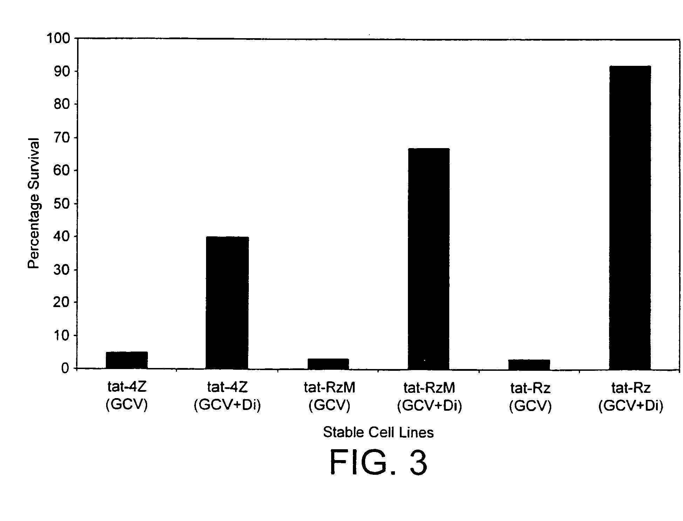

To test the feasibility of this approach we tested an anti-tat ribozyme on a tat stable cell line. As shown in Figure 3, it is possible to select for cells that contain the functional ribozyme. It is also clear that the bystander effect has to be eliminated to be able to perform such a selection.

This technique could be used to find ribozymes specific for all parts of the HIV genome. In addition this method provides a means to isolate *in vivo* relevant ribozymes for any RNA target.

### Reference Example 1 - Construction of a genome carrying a ribozyme

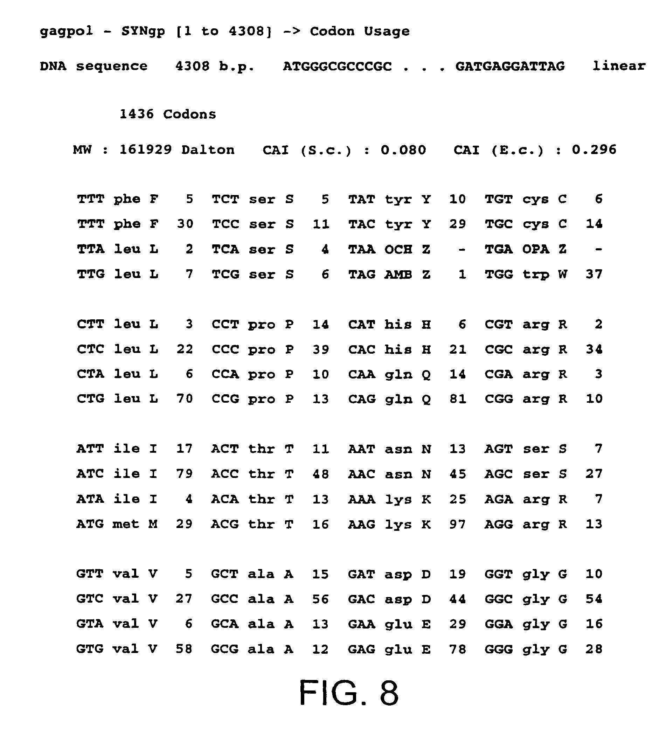

The HIV *gag.pol* sequence was codon optimised (Figure 8 and SEQ I.D. No. 1) and synthesised using overlapping oligos of around 40 nucleotides. This has three advantages. Firstly it allows an HIV based vector to carry ribozymes and other therapeutic factors. Secondly the codon optimisation generates a higher vector titre due to a higher level of gene expression. Thirdly *gag.pol* expression becomes *rev* independent which allows the use of anti-*rev* or RRE factors.

Conserved sequences within *gag.pol* were identified by reference to the HIV Sequence database at Los Alamos National Laboratory (http:// hiv-web.lanl.gov/) and used to design ribozymes. Because of the variability between subtypes of HIV-1 the ribozymes were designed to cleave the predominant subtype within North America, Latin America and the Caribbean, Europe, Japan and Australia; that is subtype B. The sites chosen were cross-referenced with the synthetic *gagpol* sequence to ensure that there was a low possibility of cutting the codon optimised gagpol mRNA. The ribozymes were designed with *Xho*I and *Sal*I sites at the 5' and 3' end respectively. This allows the construction of separate and tandem ribozymes.

The ribozymes are hammerhead structures of the following general structure:

| | | |
|---|---|---|
| Helix I | Helix II | Helix III |
| 5' - NNNNNNNN~ | CUGAUGAGGCCGAAAGGCCGAA | ~NNNNNNNN~ |

The catalytic domain of the ribozyme (Helix II) can tolerate some changes without reducing catalytic turnover.

The cleavage sites, targeting *gag* and *pol,* with the essential GUX triplet (where X is any nucleotide base) are as follows:

| | |
|---|---|
| GAG 1 | 5 ' UAGUAAGAAUGUAUAGCCCUAC |
| GAG 2 | 5 ' AACCCAGAUUGUAAGACUAUUU |
| GAG 3 | 5 ' UGUUUCAAUUGUGGCAAAGAAG |
| GAG 4 | 5 ' AAAAAGGGCUGUUGGAAAUGUG |
| POL 1 | 5 ' ACGACCCCUCGUCACAAUAAAG |
| POL 2 | 5 ' GGAAUUGGAGGUUUUAUCAAAG |
| POL 3 | 5 ' AUAUUUUUCAGUUCCCUUAGAU |
| POL 4 | 5 ' UGGAUGAUUUGUAUGUAGGAUC |
| POL 5 | 5 ' CUUUGGAUGGGUUAUGAACUCC |
| POL 6 | 5 ' CAGCUGGACUGUCAAUGACAUA |
| POL 7 | 5 ' AACUUUCUAUGUAGAUGGGGCA |
| POL 8 | 5 ' AAGGCCGCCUGUUGGUGGGCAG |
| POL 9 | 5 ' UAAGACAGCAGUACAAAUGGCA |

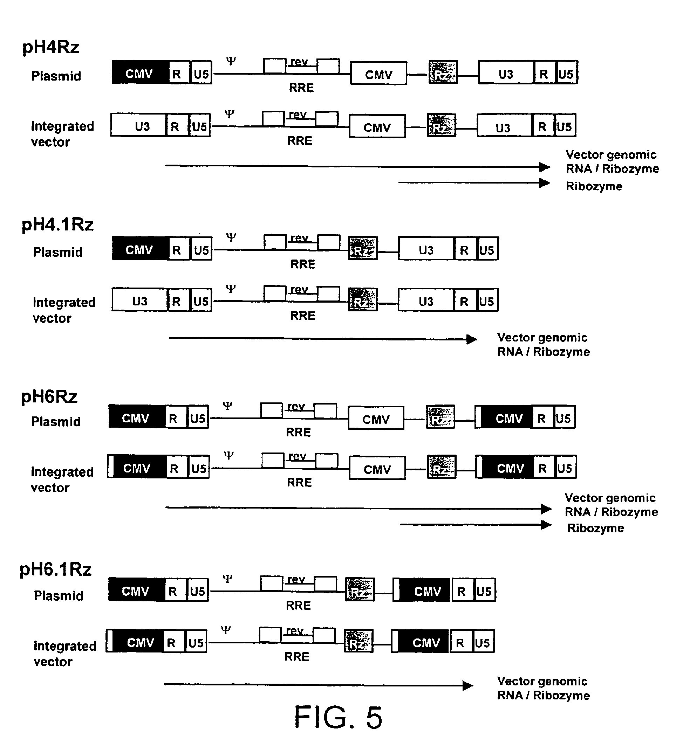

The ribozymes are inserted into four different HIV vectors (pH4 (Gervaix *et al*., 1997), pH6, pH4.1, or pH6.1) (Figure 5). In pH4 and pH6, transcription of the ribozymes is driven by an internal HCMV promoter (Foecking and Hofstetter, 1986). From pH4.1 and pH6.1, the ribozymes are expressed from the 5' LTR. The major difference between pH4 and pH6 (and pH4.1 and pH6.1) resides in the 3' LTR in the production plasmid, pH4 and pH4.1 have the HIV U3 in the 3' LTR. pH6 and pH6.1 have HCMV in the 3'LTR. The HCMV promoter replaces most of the U3 and will drive expression at high constitutive levels while the HIV-1 U3 will support a high level of expression only in the presence of Tat.

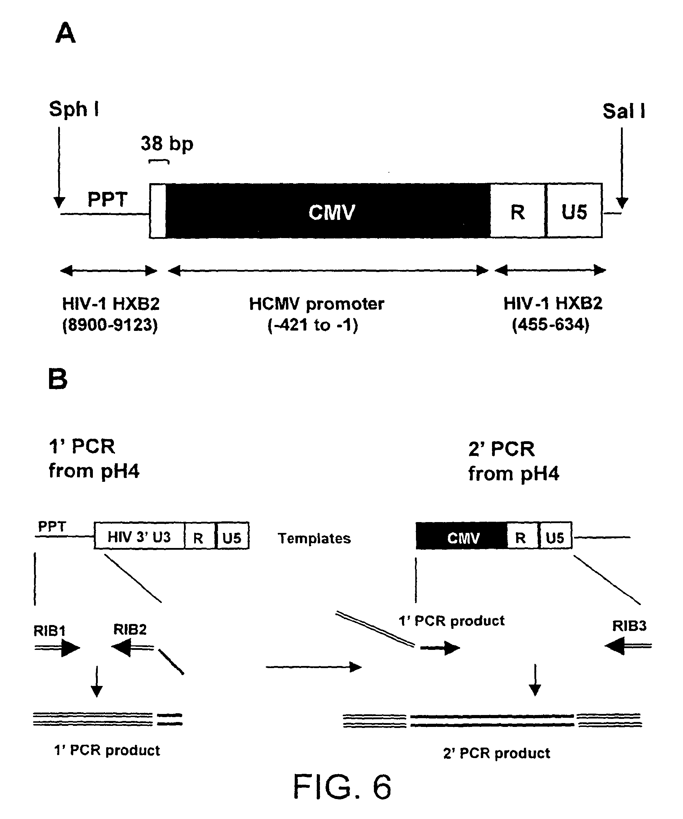

The HCMV/HIV-1 hybrid 3' LTR is created by recombinant PCR with three PCR primers (Figure 6). The first round of PCR is performed with RIB1 and RIB2 using pH4 (Kim *et al*., 1998) as the template to amplify the HIV-1 HXB2 sequence 8900-9123. The second round of PCR makes the junction between the 5' end of the HIV-1 U3 and the HCMV promoter by amplifying the hybrid 5' LTR from pH4. The PCR product from the first PCR reaction and RIB3 serves as the 5' primer and 3' primer respectively.

| | |
|---|---|
| RIB1: | 5'-CAGCTGCTCGAGCAGCTGAAGCTTGCATGC-3' |
| RIB2: | 5'-GTAAGTTATGTAACGGACGATATCTTGTCTTCTT-3' |
| RIB3: | 5'-CGCATAGTCGACGGGCCCGCCACTGCTAGAGATTTTC-3' |

The PCR product is then cut with *Sph*I and *Sal*I and inserted into pH4 thereby replacing the 3' LTR. The resulting plasmid is designated pH6. To construct pH4.1 and pH6.1, the internal HCMV promoter *(Spe*I *- Xho*I*)* in pH4 and pH6 is replaced with the polycloning site of pBluescript II KS+ (Stratagene) *(Spe*I *- Xho*I*)*.

The ribozymes are inserted into the *Xho*I sites in the genome vector backbones. Any ribozymes in any configuration could be used in a similar way.

### Reference Example 2 - Construction of a Packaging System

The packaging system can take various forms. In a first form of packaging system, the HIV gag, pol components are co-expressed with the HIV env coding sequence. In this case, both the gag, pol and the env coding sequences are altered such that they are resistant to the anti-HIV ribozymes that are built into the genome. At the same time as altering the codon usage to achieve resistance, the codons can be chosen to match the usage pattern of the most highly expressed mammalian genes. This dramatically increases expression levels (Schneider *et al*., 1997; Schwartz *et al*., 1992) and so increases titre. A codon optimised HIV env coding sequence has been described by Haas *et al*. (1996). In the present example, a modified codon optimised HIV env sequence is used (SEQ I.D. No. 3). The corresponding env expression plasmid is designated pSYNgp160mn. The modified sequence contains extra motifs not used by Haas *et al*. The extra sequences were taken from the HIV env sequence of strain MN and codon optimised. Any similar modification of the nucleic acid sequence would function similarly as long as it used codons corresponding to abundant tRNAs (Zolotukhin *et al*., 1996) and lead to resistance to the ribozymes in the genome.

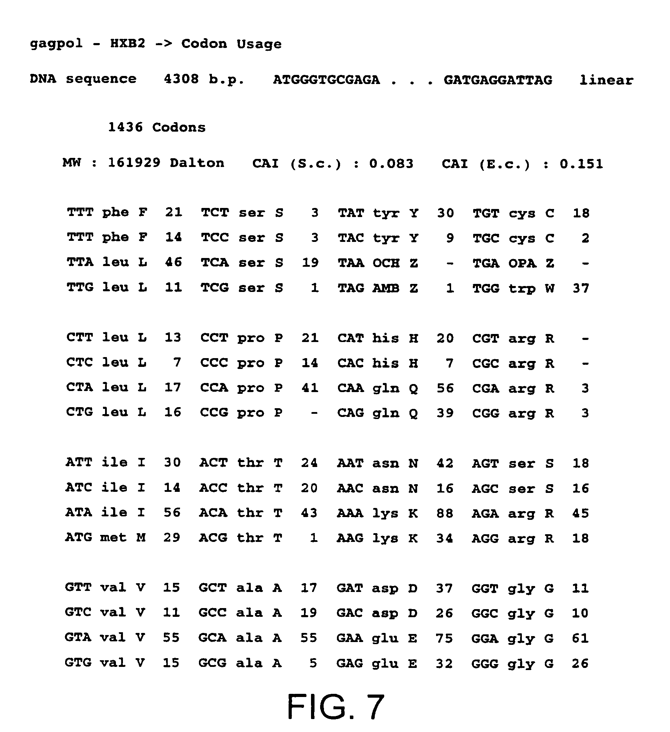

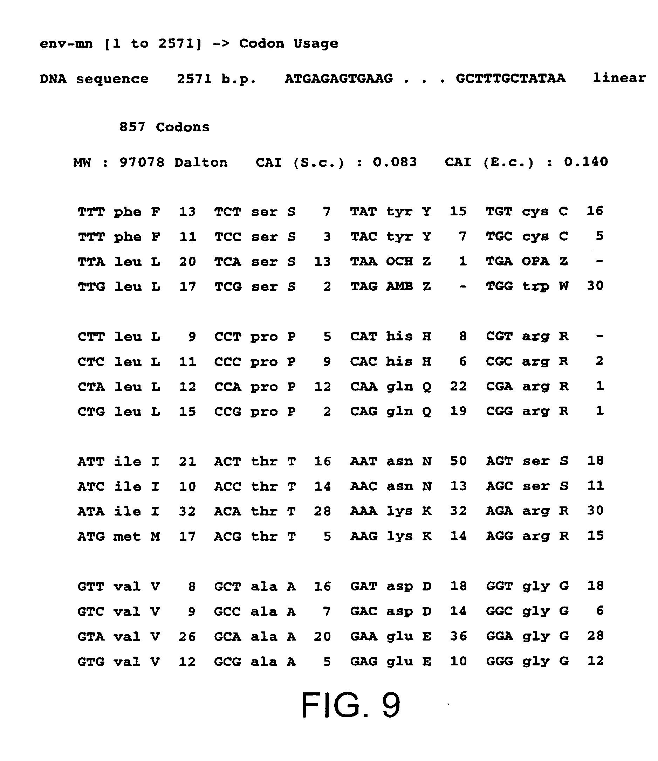

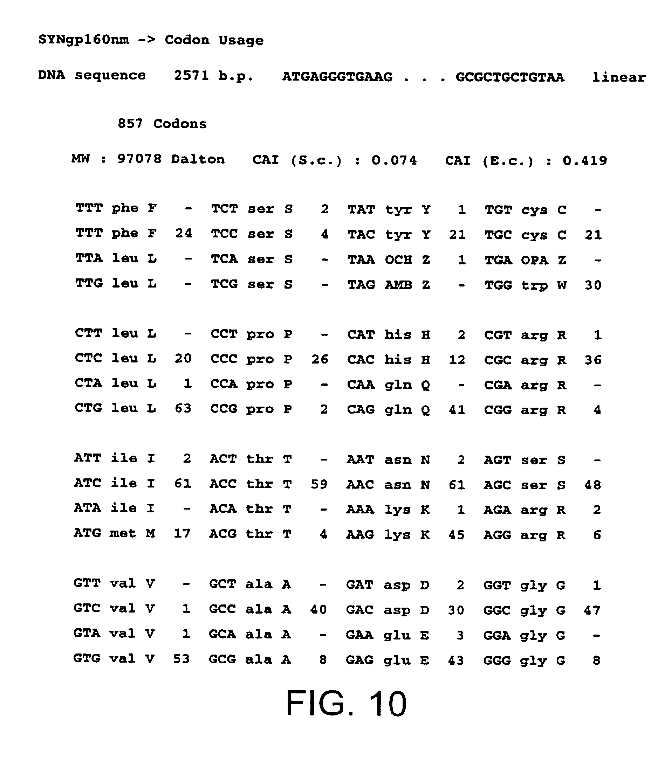

In one example of a gag, pol coding sequence with optimised codon usage, overlapping oligonucleotides are synthesised and then ligated together to produce the synthetic coding sequence. The sequence of a wild-type (Genbank accession no. K03455) and synthetic (gagpol-SYNgp) gagpol sequence is shown in SEQ I.D. Nos 1 and 2, respectively and their codon usage is shown in Figures 7 and 8, respectively. The sequence of a wild type env coding sequence (Genbank Accession No. M17449) is given in SEQ I.D. No 3, the sequence of a synthetic codon optimised sequence is given in SEQ. I.D. No. 4 and their codon usage tables are given in Figures 9 and 10, respectively. As with the env coding sequence any gag, pol sequence that achieves resistance to the ribozymes could be used. The synthetic sequence shown is designated gag, pol-SYNgp and has an *EcoR*I site at the 5' end and a *Not*I site at the 3' end. It is inserted into pClneo (Promega) to produce plasmid pSYNgp.

In a second form of the packaging system a synthetic gag, pol cassette is coexpressed with a non-HIV envelope coding sequence that produces a surface protein that pseudotypes HIV. This could be for example VSV-G (Ory *et al*., 1996: Zhu *et al.,* 1990), amphotropic MLV env (Chesebro *et al*., 1990, Spector *et al*., 1990) or any other protein that would be incorporated into the HIV particle (Valsesia Wittmann *et al.,* 1994). This includes molecules capable of targeting the vector to specific tissues. Coding sequences for non-HIV envelope proteins not cleaved by the ribozymes and so no sequence modification is required (although some sequence modification may be desirable for other reasons such as optimisation for codon usage in mammalian cells).

### Reference Example 3 - Vector Particle Production

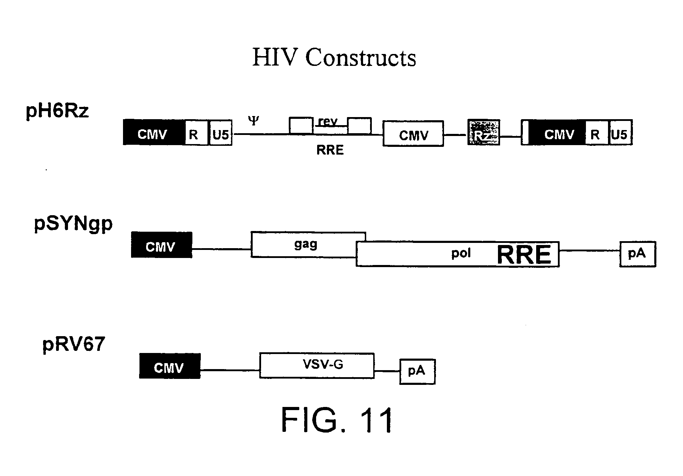

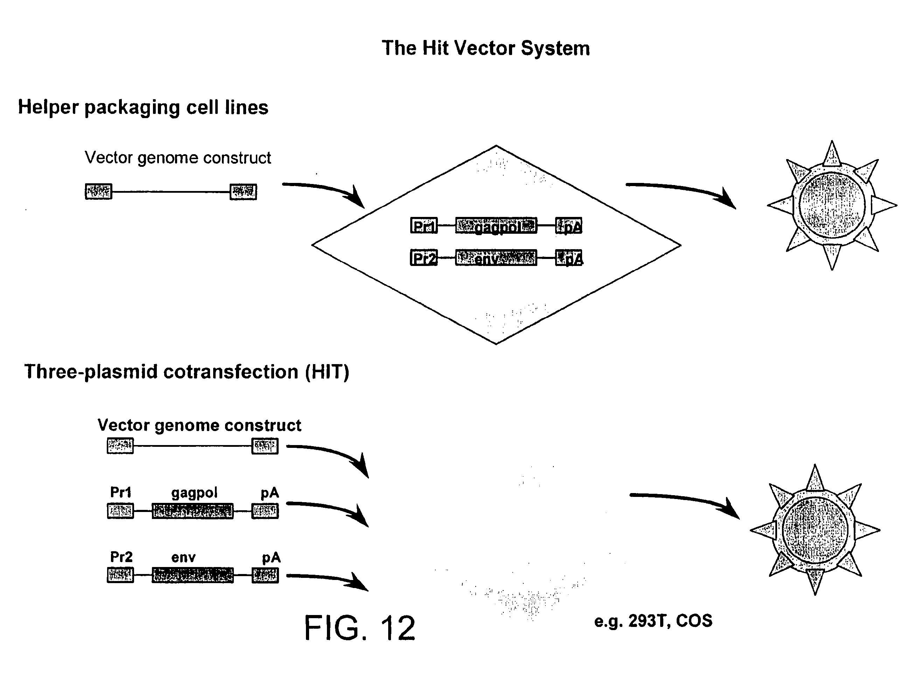

Vector particles can be produced either from a transient three-plasmid transfection system similar to that described by Soneoka *et al*. (1995) or from producer cell lines similar to those used for other retroviral vectors (Ory *et al*., 1996: Srinivasakumar *et al*., 1997; Yu *et al*., 1996). These principles are illustrated in Figures 11 and 12. For example, by using pH6Rz, pSYNgp and pRV67 (VSV-G expression plasmid) in a three plasmid transfection of 293T cells (Figure 12), as described by Soneoka *et al.* (1995), vector particles designated H6Rz-VSV are produced. These transduce the H6Rz genome to CD4+ cells such as C 1866 or Jurkat and produce the multitarget ribozymes. HIV replication in these cells is now severely restricted.

### SEQUENCE LISTING PART OF THE DESCRIPTION

SEQ. ID. NO. 1 - Wild type gagpol sequence for strain HXB2 (accession no. K03455)

SEQ I.D. NO. 2 - gagpol-SYNgp - codon optimised gagpol sequence

SEQ. ID. NO. 3 - Envelope Gene from HIV-1 MN (Genbank accession no. M17449)

SEQ. I.D. NO. 4 - SYNgp-160mn - codon optimised env sequence

### References

Bender *et al*., 1987, J Virol 61: 1639-1646
Chesebro, B.. K. Wehrly, and W. Maury. 1990. J Virol. 64:4553-7.
Foecking, M. K., and H. Hofstetter. 1986. Gene. 45:101-105.
Gervaix, A., X. Li, G. Kraus, and F. Wong Staal. 1997. J Virol. 71 :3048-53.
Haas, J., E.-C. Park, and B. Seed. 1996. Current Biology. 6:315.
Kawa *et al*., 1998, RNA 4: 1397-1406.
Kim, V. N., K. Mitrophanous, S. M. Kingsman, and K. A. J. 1998. J Virol 72: 811-816.
Ma *et al*., 1998, Antisense and Nucleic Acid Drug Development 8: 415-426.
Miller, N., and J. Whelan. 1997. Hum Gene Ther. 8:803-15.
Milner N, Mir KU, Southern EM, 1997, Nat Biotechnol., 15(6):537-41.
Ory, D. S., B. A. Neugeboren, and R. C. Mulligan. 1996. Proc Natl Acad Sci U S A. 93:11400-6.
Schneider, R., M. Campbell, G. Nasioulas, B. K. Felber, and G. N. Pavlakis. 1997. J Virol. 71 :4892-903.
Schwartz, S., M. Campbell, G. Nasioulas, J. Harrison, B. K. Felber, and G. N. Pavlakis. 1992. J Virol. 66:7176-82.
Soneoka, Y., P. M. Cannon, E. E. Ramsdale, J. C. Griffiths, G. Romano, S. M. Kingsman, and A. J. Kingsman. 1995. Nucleic Acids Res. 23:628-33.
Southern E. M., Milner N., Mir K. U., 1997, Ciba Found Symp, 209: 38-44; discussion 44-46.
Spector, D. H., E. Wade, D. A. Wright. V. Koval, C. Clark, D. Jaquish, and S. A. Spector. 1990. J Virol. 64:2298-2308.
Srinivasakumar, N., N. Chazal, C. Helga Maria, S. Prasad, M. L. Hammarskjold, and D. Rekosh. 1997. J Virol. 71 :5841-8.
Touraine RL, Ishii-Morita H, Ramsey WJ. Blaese RM, 1998, Gene Ther, 5(12):1705-11
Valsesia Wittmann, S., A. Drynda, G. Deleage, M. Aumailley, J. M. Heard, O. Danos, G. Verdier, and F. L. Cosset. 1994. J Virol. 68:4609-19.
Wagner MJ, Sharp JA, Summers WC., 1981 Proc Natl Acad Sci USA, 78(3): 1441-5. (Accession number: J02224 or V00467)
Werner *et al*., 1997, Nucleic Acids Symposium Series No. 36: 19-21.
Werner *et al*., 1998, RNA 4: 847-855.
Yu. H.. A. B. Rabson, M. Kaul. Y. Ron, and J. P. Dougherty, 1996. J Virol. 70:4530-37.
Zhou, C.. I. Bahner, J. J. Rossi. and D. B. Kohn. 1996. Antisense Nucleic Acid Drug Dev. 6:17-24.
Zhu, Z. H., S. S. Chen, and A. S. Huang. 1990.. J Acquir Immune Defic Syndr. 3:215-
Zolotukhin, S., M. Potter, W. W. Hauswirth, J. Guy, and N. Muzyczka. 1996. J Virol. 70:4646-54.

## Claims

1. A selection system suitable for use *in vivo* for selecting inhibitory RNA molecules, the system comprising:
(i) a plurality of first nucleotide sequences encoding a gene product capable of binding to and effecting the cleavage, directly or indirectly, of a nucleotide sequence or a transcription product thereof;
wherein a region of the first nucleotide sequence required for binding to the nucleotide sequence is heterogeneous within the plurality of first nucleotide sequences; and
(ii) a second nucleotide sequence comprising
(a) a coding region encoding a detectable marker operably linked to sequences required for mRNA stability and/or translation; and
(b) a third nucleotide sequence positioned between the coding region and at least one of the sequences required for mRNA stability and/or translation;
wherein (a) and (b) are operably linked to a regulatory sequence capable of directing expression of (a) and (b) as a contiguous RNA molecule in a host cell; and wherein the gene product encoded by the first nucleotide sequence is capable of binding to an effecting the cleavage, directly or indirectly, of the third nucleotide sequence or a transcription product thereof.

2. A vector system for selecting inhibitory RNA molecules comprising:
(i) a plurality of vectors, each vector independently comprising
a first nucleotide sequence encoding a gene product capable of binding to and effecting the cleavage, directly or indirectly, of a nucleotide sequence or a transcription product thereof;
wherein a region of the first nucleotide sequence required for binding to the nucleotide sequence is heterogeneous within the plurality of vectors; and
(ii) a second nucleotide sequence comprising
(a) a coding region encoding a detectable marker operably linked to sequences required for mRNA stability and/or translation; and
(b) a third nucleotide sequence positioned between the coding region and at least one of the sequences required for mRNA stability and/or translation;
wherein (a) and (b) are operably linked to a regulatory sequence capable of directing expression of (a) and (b) as a contiguous RNA molecule in a host cell,
wherein the second nucleotide sequence is present in the plurality of vectors or as part of a separate vector;
and wherein the gene product encoded by the first nucleotide sequence is capable of binding to and effecting the cleavage, directly or indirectly, of the third nucleotide sequence or a transcription product thereof.

3. A system according to claim 1 or 2 wherein the gene product is selected from a ribozyme, an anti-sense ribonucleic acid and an external guide sequence.

4. A system according to claim 2 or 3 wherein the system is a system according to claim 2 and wherein the vector is a viral vector.

5. A system according to claim 4 wherein the viral vector is a retroviral vector.

6. A system according to any one of the preceding claims wherein the detectable marker is a selectable marker.

7. A system according to claim 6 wherein the selectable marker is an enzyme capable of converting a prodrug into a cytotoxic compound.

8. A system according to claim 7 wherein the enzyme is thymidine kinase and the prodrug is gancyclovir.

9. A system according to any one of the preceding claims wherein the third nucleotide sequence is present in the 3' and/or 5' untranslated region of the second nucleotide sequence.

10. A plurality of viral particles, each viral particle comprising a first nucleotide sequence according to claim 1 and a second nucleotide sequence according to claim 1.

11. A method for producing plurality of viral particles according to claim 10 which method comprises introducing into a producer cell (i) a plurality of first nucleotide sequences as defined in claim 1 and (ii) a second nucleotide sequence as defined in claim 1.

12. A plurality of viral particles produced by the method of claim 11.

13. A method for selecting from a plurality of first nucleotide sequences, a first nucleotide sequence encoding a gene product capable of binding to and effecting the cleavage, directly or indirectly, of a third nucleotide sequence or transcription product thereof; which method comprises:
(i) introducing the system according to claim 1 or claim 2 into a host cell; and
(ii) selecting the host cell if the detectable marker is not expressed in an active form in the host cell; and optionally,
(iii) isolating the first nucleotide sequence and determining its nucleotide sequence.

14. A method according to claim 13 wherein in step (ii), the detectable marker is a selectable marker and the host cell is contacted with a compound which is cytotoxic in the presence of the selectable marker.

15. A method according to claim 14 wherein the selectable marker is thymidine kinase and the compound is gancyclovir.

16. A method according to any one of claims 13 to 15, which further comprises a selection step to remove any first nucleotide sequence(s) encoding a gene product capable of binding to and effecting the cleavage directly or indirectly of a coding region encoding the detectable marker.

17. A method according to claim 16, wherein each first nucleotide sequence in the plurality of first nucleotide sequences is located downstream of a fourth nucleotide sequence encoding an inactive variant of the detectable marker.

18. A method according to claim 16 or 17, wherein the plurality of first nucleotide sequences are present in a plurality of viral vectors, said viral vectors comprising a fourth nucleotide sequence encoding an inactive variant of the detectable marker, and as a preliminary step, the plurality of vectors are introduced into one or more producer cells and any resulting infectious viral particles are used in step (i) of the selection method of any of claims 13 to 15.

19. A method for identifying an open site on a mRNA molecule, using a system according to any of claims 1 to 9.

## Patentansprüche

1. Selektionssystem, das für eine Verwendung *in vivo* zum Selektieren von inhibitorischen RNA-Molekülen geeignet ist, wobei das System folgendes umfaßt:
(i) eine Mehrzahl erster Nukleotidsequenzen, die ein Genprodukt codieren, welches in der Lage ist, an eine Nukleotidsequenz oder ein Transkriptionsprodukt davon zu binden und direkt oder indirekt dessen Spaltung zu bewirken,
wobei eine Region der ersten Nukleotidsequenz, die für eine Bindung an die Nukleotidsequenz erforderlich ist, innerhalb der Mehrzahl von ersten Nukleotidsequenzen heterogen ist, und
(ii) eine zweite Nukleotidsequenz mit
(a) einer codierenden Region, die einen detektierbaren Marker codiert, der funktionsfähig mit Sequenzen verknüpft ist, die für mRNA-Stabilität und/oder -Translation erforderlich sind, und
(b) einer dritten Nukleotidsequenz, die zwischen der codierenden Region und wenigstens einer der Sequenzen, die für mRNA-Stabilität und/oder -Translation erforderlich sind, angeordnet ist,
wobei (a) und (b) funktionsfähig mit einer regulatorischen Sequenz verknüpft sind, die in der Lage ist, die Expression von (a) und (b) als ein zusammenhängendes RNA-Molekül in einer Wirtszelle zu steuern, und wobei das von der ersten Nukleotidsequenz codierte Genprodukt in der Lage ist, an die dritte Nukleotidsequenz oder ein Transkriptionsprodukt davon zu binden und direkt oder indirekt dessen Spaltung zu bewirken.

2. Vektorsystem zum Selektieren von inhibitorischen RNA-Molekülen mit
(i) einer Mehrzahl von Vektoren, wobei jeder Vektor unabhängig voneinander folgendes umfaßt:
eine erste Nukleotidsequenz, die ein Genprodukt codiert, das in der Lage ist, an eine Nukleotidsequenz oder ein Transkriptionsprodukt davon zu binden und direkt oder indirekt dessen Spaltung zu bewirken,
wobei eine Region der ersten Nukleotidsequenz, die zur Bindung an die Nukleotidsequenz erforderlich ist, innerhalb der Mehrzahl von Vektoren heterogen ist, und
(ii) eine zweite Nukleotidsequenz mit
(a) einer codierenden Region, die einen detektierbaren Marker codiert, der funktionsfähig mit Sequenzen verknüpft ist, die für mRNA-Stabilität und/oder -Translation erforderlich sind, und
(b) einer dritten Nukleotidsequenz, die zwischen der codierenden Region und wenigstens einer der Sequenzen, die für mRNA-Stabilität und/oder -Translation erforderlich sind, angeordnet ist,
wobei (a) und (b) funktionsfähig mit einer regulatorischen Sequenz verknüpft sind, welche in der Lage ist, die Expression von (a) und (b) als ein zusammenhängendes RNA-Molekül in einer Wirtszelle zu steuern,
wobei die zweite Nukleotidsequenz in der Mehrzahl von Vektoren oder als ein Teil eines separaten Vektors vorliegt
und wobei das von der ersten Nukleotidsequenz codierte Genprodukt in der Lage ist, an die dritte Nukleotidsequenz oder ein Transkriptionsprodukt davon zu binden und direkt oder indirekt dessen Spaltung zu bewirken.

3. System nach Anspruch 1 oder 2, wobei das Genprodukt unter einem Ribozym, einer Antisense-Ribonukleinsäure und einer externen Führungssequenz ausgewählt ist.

4. System nach Anspruch 2 oder 3, wobei das System ein System nach Anspruch 2 ist und wobei der Vektor ein viraler Vektor ist.

5. System nach Anspruch 4, wobei der virale Vektor ein retroviraler Vektor ist.

6. System nach einem der vorangegangenen Ansprüche, wobei der detektierbare Marker ein selektierbarer Marker ist.

7. System nach Anspruch 6, wobei der selektierbare Marker ein Enzym ist, welches in der Lage ist, einen Wirkstoffvorläufer in eine cytotoxische Verbindung umzuwandeln.

8. System nach Anspruch 7, wobei das Enzym Thymidinkinase ist und der Wirkstoffvorläufer Gancyclovir ist.

9. System nach einem der vorangegangenen Ansprüche, wobei die dritte Nukleotidsequenz in der 3'- und/oder 5'-untranslatierten Region der zweiten Nukleotidsequenz vorhanden ist.

10. Mehrzahl von viralen Partikeln, wobei jedes virale Partikel eine erste Nukleotidsequenz nach Anspruch 1 und eine zweite Nukleotidsequenz nach Anspruch 1 umfaßt.

11. Verfahren zur Herstellung einer Mehrzahl viraler Partikel nach Anspruch 10, wobei das Verfahren das Einbringen von (i) einer Mehrzahl erster Nukleotidsequenzen, wie sie in Anspruch 1 definiert sind, und (ii) einer zweiten Nukleotidsequenz, wie sie in Anspruch 1 definiert ist, in eine Herstellerzelle umfaßt.

12. Mehrzahl viraler Partikel, hergestellt nach dem Verfahren von Anspruch 11.

13. Verfahren zum Selektieren einer ersten Nukleotidsequenz, die ein Genprodukt codiert, welches in der Lage ist, an eine dritte Nukleotidsequenz oder ein Transkriptionsprodukt davon zu binden und direkt oder indirekt dessen Spaltung zu bewirken, unter einer Mehrzahl von ersten Nukleotidsequenzen, wobei das Verfahren folgendes umfaßt:
(i) Einbringen des Systems nach Anspruch 1 oder Anspruch 2 in eine Wirtszelle und
(ii) Auswählen der Wirtszelle, wenn der detektierbare Marker in der Wirtszelle nicht in einer aktiven Form exprimiert wird, und optional
(iii) Isolieren der ersten Nukleotidsequenz und Bestimmen von dessen Nukleotidsequenz.

14. Verfahren nach Anspruch 13, wobei in Stufe (ii) der detektierbare Marker ein selektierbarer Marker ist und die Wirtszelle mit einer Verbindung in Kontakt gebracht wird, die in der Gegenwart des selektierbaren Markers cytotoxisch ist.

15. Verfahren nach Anspruch 14, wobei der selektierbare Marker Thymidinkinase ist und die Verbindung Gancyclovir ist.

16. Verfahren nach einem der Ansprüche 13 bis 15, welches weiterhin eine Selektionsstufe umfaßt, um sämtliche erste Nukleotidsequenz(en) zu entfernen, welche ein Genprodukt codiert bzw. codieren, das in der Lage ist, eine codierende Region, die den detektierbaren Marker codiert, zu binden und direkt oder indirekt dessen Spaltung zu bewirken.

17. Verfahren nach Anspruch 16, wobei jede erste Nukleotidsequenz in der Mehrzahl von ersten Nukleotidsequenzen abstromig zu einer vierten Nukleotidsequenz, die eine inaktive Variante des detektierbaren Markers codiert, angeordnet ist.

18. Verfahren nach Anspruch 16 oder 17, wobei die Mehrzahl von ersten Nukleotidsequenzen in einer Mehrzahl viraler Vektoren vorliegt, wobei die viralen Vektoren eine vierte Nukleotidsequenz umfassen, welche eine inaktive Variante des detektierbaren Markers codiert, und wobei als eine vorläufige Stufe die Mehrzahl von Vektoren in eine oder mehrere Herstellerzellen eingebracht wird und jegliche resultierende infektiöse virale Partikel in Stufe (i) des Selektionsverfahrens nach einem der Ansprüche 13 bis 15 eingesetzt werden.

19. Verfahren zum Identifizieren einer offenen Stelle auf einem mRNA-Molekül unter Verwendung eines Systems nach einem der Ansprüche 1 bis 9.

## Revendications

1. Système de sélection approprié à une utilisation *in vivo* pour sélectionner des molécules d'ARN inhibitrices, le système comprenant :
(i) une pluralité de premières séquences nucléotidiques encodant un produit génétique capable de se lier à et d'effectuer le clivage, directement ou indirectement, d'une séquence nucléotidique ou d'un produit de transcription de celle-ci ;
dans lequel une région de la première séquence nucléotidique requise pour la liaison à la séquence nucléotidique est hétérogène dans la pluralité de premières séquences nucléotidiques ; et
(ii) une deuxième séquence nucléotidique comprenant
(a) une région codante encodant un marqueur détectable opérationnellement lié aux séquences requises pour la stabilité et/ou traduction de l'ARNm ; et
(b) une troisième séquence nucléotidique positionnée entre la région codante et au moins l'une des séquences requises pour la stabilité et/ou traduction de l'ARNm ;
dans lequel (a) et (b) sont opérationnellement liées à une séquence régulatrice capable de diriger l'expression de (a) et (b) sous la forme d'une molécule d'ARN contiguë dans une cellule hôte ; et dans lequel le produit génétique encodé par la première séquence nucléotidique est capable de se lier à et d'effectuer le clivage, directement ou indirectement, de la troisième séquence nucléotidique ou d'un produit de transcription de celle-ci.

2. Système vecteur pour sélectionner des molécules d'ARN inhibitrices comprenant :
(i) une pluralité de vecteurs, chaque vecteur comprenant indépendamment
une première séquence nucléotidique encodant un produit génétique capable de se lier à et d'effectuer le clivage, directement ou indirectement, d'une séquence nucléotidique ou d'un produit de transcription de celle-ci ;
dans lequel une région de la première séquence nucléotidique requise pour la liaison à la séquence nucléotidique est hétérogène dans la pluralité de vecteurs ; et
(ii) une deuxième séquence nucléotidique comprenant
(a) une région codante encodant un marqueur détectable opérationnellement lié aux séquences requises pour la stabilité et/ou traduction de l'ARNm ; et
(b) une troisième séquence nucléotidique positionnée entre la région codante et au moins l'une des séquences requises pour la stabilité et/ou traduction de l'ARNm ;
dans lequel (a) et (b) sont opérationnellement liées à une séquence régulatrice capable de diriger l'expression de (a) et (b) sous la forme d'une molécule d'ARN contiguë dans une cellule hôte,
dans lequel la deuxième séquence nucléotidique est présente dans la pluralité de vecteurs ou fait partie d'un vecteur séparé ;
et dans lequel le produit génétique encodé par la première séquence nucléotidique est capable de se lier à et d'effectuer le clivage, directement ou indirectement, de la troisième séquence nucléotidique ou d'un produit de transcription de celle-ci.

3. Système selon la revendication 1 ou 2, dans lequel le produit génétique est choisi parmi un ribozyme, un acide ribonucléique anti-sens et une séquence guide externe.

4. Système selon la revendication 2 ou 3, lequel système est un système selon la revendication 2 et dans lequel le vecteur est un vecteur viral.

5. Système selon la revendication 4, dans lequel le vecteur viral est un vecteur rétroviral.

6. Système selon l'une quelconque des revendications précédentes, dans lequel le marqueur détectable est un marqueur sélectionnable.

7. Système selon la revendication 6, dans lequel le marqueur sélectionnable est une enzyme capable de convertir un promédicament en un composé cytotoxique.

8. Système selon la revendication 7, dans lequel l'enzyme est la thymidine kinase et le promédicament est le gancyclovir.

9. Système selon l'une quelconque des revendications précédentes, dans lequel la troisième séquence nucléotidique est présente dans la région non traduite 3' et/ou 5' de la deuxième séquence nucléotidique.

10. Pluralité de particules virales, chaque particule virale comprenant une première séquence nucléotidique selon la revendication 1 et une deuxième séquence nucléotidique selon la revendication 1.

11. Procédé de production d'une pluralité de particules virales selon la revendication 10, lequel procédé comprend l'introduction dans une cellule productrice (i) d'une pluralité de premières séquences nucléotidiques telles que définies dans la revendication 1 et (ii) d'une deuxième séquence nucléotidique telle que définie dans la revendication 1.

12. Pluralité de particules virales produites par le procédé de la revendication 11.

13. Procédé de sélection à partir d'une pluralité de premières séquences nucléotidiques, d'une première séquence nucléotidique encodant un produit génétique capable de se lier à et d'effectuer le clivage, directement ou indirectement, d'une troisième séquence nucléotidique ou d'un produit de transcription de celle-ci ; lequel procédé comprend :
(i) l'introduction du système selon la revendication 1 ou la revendication 2 dans une cellule hôte ; et
(ii) la sélection de la cellule hôte si le marqueur détectable n'est pas exprimé sous une forme active dans la cellule hôte ; et facultativement,
(iii) l'isolation de la première séquence nucléotidique et la détermination de sa séquence nucléotidique.

14. Procédé selon la revendication 13, dans lequel dans l'étape (ii), le marqueur détectable est un marqueur sélectionnable et la cellule hôte est mise en contact avec un composé qui est cytotoxique en présence du marqueur sélectionnable.

15. Procédé selon la revendication 14, dans lequel le marqueur sélectionnable est la thymidine kinase et le composé est le gancyclovir.

16. Procédé selon l'une quelconque des revendications 13 à 15, qui comprend également une étape de sélection pour éliminer toute(s) première(s) séquence(s) nucléotidique(s) encodant un produit génétique capable de se lier à et d'effectuer le clivage, directement ou indirectement, d'une région codante encodant le marqueur détectable.

17. Procédé selon la revendication 16, dans lequel chaque première séquence nucléotidique dans la pluralité de premières séquences nucléotidiques est située en aval d'une quatrième séquence nucléotidique encodant un variant inactif du marqueur détectable.

18. Procédé selon la revendication 16 ou 17, dans lequel la pluralité de premières séquences nucléotidiques est présente dans une pluralité de vecteurs viraux, lesdits vecteurs viraux comprenant une quatrième séquence nucléotidique encodant un variant inactif du marqueur détectable, et comme étape préliminaire, la pluralité de vecteurs est introduite dans une ou plusieurs cellules productrices et toutes les particules virales infectieuses résultantes sont utilisées dans l'étape (i) du procédé de sélection de l'une quelconque des revendications 13 à 15.

19. Procédé d'identification d'un site ouvert sur une molécule d'ARNm, en utilisant un système selon l'une quelconque des revendications 1 à 9.


### SEQUENCE LISTING

<110> Oxford BioMedica (UK) Limited
   <110> MITROPHANOUS, Kyri
   <110> KIM, Narry
   <110> KOTSOPOULE, Ekaterina
   <120> In Vivo Selection Method
<130> P006914WOCTH
<140> PCT/GB00/02136
   <141> 02/06/2000
<150> 9912965.2
   <151> 03/06/1999
<160> 26
<170> SeqWin99
<210> 1
   <211> 4307
   <212> DNA
   <213> Human immunodeficiency virus type I
<400> 1
   atgggtgcga gagcgtcagt attaagcggg ggagaattag atcgatggga aaaaattcgg 60
   ttaaggccag ggggaaagaa aaaatataaa ttaaaacata tagtatgggc aagcagggag 120
   ctagaacgat tcgcagttaa tcctggcctg ttagaaacat cagaaggctg tagacaaata 180
   ctgggacagc tacaaccatc ccttcagaca ggatcagaag aacttagatc attatataat 240
   acagtagcaa ccctctattg tgtgcatcaa aggatagaga taaaagacac caaggaagct 300
   ttagacaaga tagaggaaga gcaaaacaaa agtaagaaaa aagcacagca agcagcagct 360
   gacacaggac acagcaatca ggtcagccaa aattacccta tagtgcagaa catccagggg 420
   caaatggtac atcaggccat atcacctaga actttaaatg catgggtaaa agtagtagaa 480
   gagaaggctt tcagcccaga agtgataccc atgttttcag cattatcaga aggagccacc 540
   ccacaagatt taaacaccat gctaaacaca gtggggggac atcaagcagc catgcaaatg 600
   ttaaaagaga ccatcaatga ggaagctgca gaatgggata gagtgcatcc agtgcatgca 660
   gggcctattg caccaggcca gatgagagaa ccaaggggaa gtgacatagc aggaactact 720
   agtacccttc aggaacaaat aggatggatg acaaataatc cacctatccc agtaggagaa 780
   atttataaaa gatggataat cctgggatta aataaaatag taagaatgta tagccctacc 840
   agcattctgg acataagaca aggaccaaag gaacccttta gagactatgt agaccggttc 900
   tataaaactc taagagccga gcaagcttca caggaggtaa aaaattggat gacagaaacc 960
   ttgttggtcc aaaatgcgaa cccagattgt aagactattt taaaagcatt gggaccagcg 1020
   gctacactag aagaaatgat gacagcatgt cagggagtag gaggacccgg ccataaggca 1080
   agagttttgg ctgaagcaat gagccaagta acaaattcag ctaccataat gatgcagaga 1140
   ggcaatttta ggaaccaaag aaagattgtt aagtgtttca attgtggcaa agaagggcac 1200
   acagccagaa attgcagggc ccctaggaaa aagggctgtt ggaaatgtgg aaaggaagga 1260
   caccaaatga aagattgtac tgagagacag gctaattttt tagggaagat ctggccttcc 1320
   tacaagggaa ggccagggaa ttttcttcag agcagaccag agccaacagc cccaccagaa 1380
   gagagcttca ggtctggggt agagacaaca actccccctc agaagcagga gccgatagac 1440
   aaggaactgt atcctttaac ttccctcagg tcactctttg gcaacgaccc ctcgtcacaa 1500
   taaagatagg ggggcaacta aaggaagctc tattagatac aggagcagat gatacagtat 1560
   tagaagaaat gagtttgcca ggaagatgga aaccaaaaat gataggggga attggaggtt 1620
   ttatcaaagt aagacagtat gatcagatac tcatagaaat ctgtggacat aaagctatag 1680
   gtacagtatt agtaggacct acacctgtca acataattgg aagaaatctg ttgactcaga 1740
   ttggttgcac tttaaatttt cccattagcc ctattgagac tgtaccagta aaattaaagc 1800
   caggaatgga tggcccaaaa gttaaacaat ggccattgac agaagaaaaa ataaaagcat 1860
   tagtagaaat ttgtacagag atggaaaagg aaggaaaaat ttcaaaaatt gggcctgaaa 1920
   atccatacaa tactccagta tttgccataa agaaaaaaga cagtactaaa tggagaaaat 1980
   tagtagattt cagagaactt aataagagaa ctcaagactt ctgggaagtt caattaggaa 2040
   taccacatcc cgcagggtta aaaaagaaaa aatcagtaac agtactggat gtgggtgatg 2100
   catatttttc agttccctta gatgaagact tcaggaagta tactgcattt accataccta 2160
   gtataaacaa tgagacacca gggattagat atcagtacaa tgtgcttcca cagggatgga 2220
   aaggatcacc agcaatattc caaagtagca tgacaaaaat cttagagcct tttagaaaac   2280
   aaaatccaga catagttatc tatcaataca tggatgattt gtatgtagga tctgacttag   2340
   aaatagggca gcatagaaca aaaatagagg agctgagaca acatctgttg aggtggggac   2400
   ttaccacacc agacaaaaaa catcagaaag aacctccatt cctttggatg ggttatgaac   2460
   tccatcctga taaatggaca gtacagccta tagtgctgcc agaaaaagac agctggactg   2520
   tcaatgacat acagaagtta gtggggaaat tgaattgggc aagtcagatt tacccaggga   2580
   ttaaagtaag gcaattatgt aaactcctta gaggaaccaa agcactaaca gaagtaatac   2640
   cactaacaga agaagcagag ctagaactgg cagaaaacag agagattcta aaagaaccag   2700
   tacatggagt gtattatgac ccatcaaaag acttaatagc agaaatacag aagcaggggc   2760
   aaggccaatg gacatatcaa atttatcaag agccatttaa aaatctgaaa acaggaaaat    2820
   atgcaagaat gaggggtgcc cacactaatg atgtaaaaca attaacagag gcagtgcaaa    2880
   aaataaccac agaaagcata gtaatatggg gaaagactcc taaatttaaa ctgcccatac   2940
   aaaaggaaac atgggaaaca tggtggacag agtattggca agccacctgg attcctgagt   3000
   gggagtttgt taatacccct cccttagtga aattatggta ccagttagag aaagaaccca   3060
   tagtaggagc agaaaccttc tatgtagatg gggcagctaa cagggagact aaattaggaa   3120
   aagcaggata tgttactaat agaggaagac aaaaagttgt caccctaact gacacaacaa   3180
   atcagaagac tgagttacaa gcaatttatc tagctttgca ggattcggga ttagaagtaa   3240
   acatagtaac agactcacaa tatgcattag gaatcattca agcacaacca gatcaaagtg   3300
   aatcagagtt agtcaatcaa ataatagagc agttaataaa aaaggaaaag gtctatctgg   3360
   catgggtacc agcacacaaa ggaattggag gaaatgaaca agtagataaa ttagtcagtg   3420
   ctggaatcag gaaagtacta tttttagatg gaatagataa ggcccaagat gaacatgaga   3480
   aatatcacag taattggaga gcaatggcta gtgattttaa cctgccacct gtagtagcaa   3540
   aagaaatagt agccagctgt gataaatgtc agctaaaagg agaagccatg catggacaag   3600
   tagactgtag tccaggaata tggcaactag attgtacaca tttagaagga aaagttatcc   3660
   tggtagcagt tcatgtagcc agtggatata tagaagcaga agttattcca gcagaaacag   3720
   ggcaggaaac agcatatttt cttttaaaat tagcaggaag atggccagta aaaacaatac   3780
   atactgacaa tggcagcaat ttcaccggtg ctacggttag ggccgcctgt tggtgggcgg   3840
   gaatcaagca ggaatttgga attccctaca atccccaaag tcaaggagta gtagaatcta   3900
   tgaataaaga attaaagaaa attataggac aggtaagaga tcaggctgaa catcttaaga   3960
   cagcagtaca aatggcagta ttcatccaca attttaaaag aaaagggggg attggggggt   4020
   acagtgcagg ggaaagaata gtagacataa tagcaacaga catacaaact aaagaattac   4080
   aaaaacaaat tacaaaaatt caaaattttc gggtttatta cagggacagc agaaattcac   4140
   tttggaaagg accagcaaag ctcctctgga aaggtgaagg ggcagtagta atacaagata   4200
   atagtgacat aaaagtagtg ccaagaagaa aagcaaagat cattagggat tatggaaaac   4260
   agatggcagg tgatgattgt gtggcaagta gacaggatga ggattag                 4307
<210> 2
   <211> 4307
   <212> DNA
   <213> Artificial Sequence
<220>
   <223> Codon optimised gagpol sequence
<400> 2
   atgggcgccc gcgccagcgt gctgtcgggc ggcgagctgg accgctggga gaagatccgc   60
   ctgcgccccg gcggcaaaaa gaagtacaag ctgaagcaca tcgtgtgggc cagccgcgaa  120
   ctggagcgct tcgccgtgaa ccccgggctc ctggagacca gcgaggggtg ccgccagatc  180
   ctcggccaac tgcagcccag cctgcaaacc ggcagcgagg agctgcgcag cctgtacaac  240
   accgtggcca cgctgtactg cgtccaccag cgcatcgaaa tcaaggatac gaaagaggcc  300
   ctggataaaa tcgaagagga acagaataag agcaaaaaga aggcccaaca ggccgccgcg  360
   gacaccggac acagcaacca ggtcagccag aactacccca tcgtgcagaa catccagggg  420
   cagatggtgc accaggccat ctccccccgc acgctgaacg cctgggtgaa ggtggtggaa  480
   gagaaggctt ttagcccgga ggtgataccc atgttctcag ccctgtcaga gggagccacc  540
   ccccaagatc tgaacaccat gctcaacaca gtggggggac accaggccgc catgcagatg  600
   ctgaaggaga ccatcaatga ggaggctgcc gaatgggatc gtgtgcatcc ggtgcacgca  660
   gggcccatcg caccgggcca gatgcgtgag ccacggggct cagacatcgc cggaacgact  720
   agtacccttc aggaacagat cggctggatg accaacaacc cacccatccc ggtgggagaa  780
   atctacaaac gctggatcat cctgggcctg aacaagatcg tgcgcatgta tagccctacc  840
   agcatcctgg acatccgcca aggcccgaag gaaccctttc gcgactacgt ggaccggttc  900
   tacaaaacgc tccgcgccga gcaggctagc caggaggtga agaactggat gaccgaaacc  960
   ctgctggtcc agaacgcgaa cccggactgc aagacgatcc tgaaggccct gggcccagcg 1020
   gctaccctag aggaaatgat gaccgcctgt cagggagtgg gcggacccgg ccacaaggca 1080
   cgcgtcctgg ctgaggccat gagccaggtg accaactccg ctaccatcat gatgcagcgc 1140
   ggcaactttc ggaaccaacg caagatcgtc aagtgcttca actgtggcaa agaagggcac 1200
   acagcccgca actgcagggc ccctaggaaa aagggctgct ggaaatgcgg caaggaaggc 1260
   caccagatga aagactgtac tgagagacag gctaattttt tagggaagat ctggccttcc 1320
   tacaagggaa ggccagggaa ttttcttcag agcagaccag agccaacagc cccaccagaa 1380
   gagagcttca ggtctggggt agagacaaca actccccctc agaagcagga gccgatagac 1440
   aaggaactgt atcctttaac ttccctcaga tcactctttg gcaacgaccc ctcgtcacaa 1500
   taaagatagg ggggcagctc aaggaggctc tcctggacac cggagcagac gacaccgtgc 1560
   tggaggagat gtcgttgcca ggccgctgga agccgaagat gatcggggga atcggcggtt 1620
   tcatcaaggt gcgccagtat gaccagatcc tcatcgaaat ctgcggccac aaggctatcg 1680
   gtaccgtgct ggtgggcccc acacccgtca acatcatcgg acgcaacctg ttgacgcaga 1740
   tcggttgcac gctgaacttc cccattagcc ctatcgagac ggtaccggtg aagctgaagc 1800
   ccgggatgga cggcccgaag gtcaagcaat ggccattgac agaggagaag atcaaggcac 1860
   tggtggagat ttgcacagag atggaaaagg aagggaaaat ctccaagatt gggcctgaga 1920
   acccgtacaa cacgccggtg ttcgcaatca agaagaagga ctcgacgaaa tggcgcaagc 1980
   tggtggactt ccgcgagctg aacaagcgca cgcaagactt ctgggaggtt cagctgggca 2040
   tcccgcaccc cgcagggctg aagaagaaga aatccgtgac cgtactggat gtgggtgatg 2100
   cctacttctc cgttcccctg gacgaagact tcaggaagta cactgccttc acaatccctt 2160
   cgatcaacaa cgagacaccg gggattcgat atcagtacaa cgtgctgccc cagggctgga 2220
   aaggctctcc cgcaatcttc cagagtagca tgaccaaaat cctggagcct ttccgcaaac 2280
   agaaccccga catcgtcatc tatcagtaca tggatgactt gtacgtgggc tctgatctag 2340
   agatagggca gcaccgcacc aagatcgagg agctgcgcca gcacctgttg aggtggggac 2400
   tgaccacacc cgacaagaag caccagaagg agcctccctt cctctggatg ggttacgagc 2460
   tgcaccctga caaatggacc gtgcagccta tcgtgctgcc agagaaagac agctggactg 2520
   tcaacgacat acagaagctg gtggggaagt tgaactgggc cagtcagatt tacccaggga 2580
   ttaaggtgag gcagctgtgc aaactcctcc gcggaaccaa ggcactcaca gaggtgatcc 2640
   ccctaaccga ggaggccgag ctcgaactgg cagaaaaccg agagatccta aaggagcccg 2700
   tgcacggcgt gtactatgac ccctccaagg acctgatcgc cgagatccag aagcaggggc 2760
   aaggccagtg gacctatcag atttaccagg agcccttcaa gaacctgaag accggcaagt 2820
   acgcccggat gaggggtgcc cacactaacg acgtcaagca gctgaccgag gccgtgcaga 2880
   agatcaccac cgaaagcatc gtgatctggg gaaagactcc taagttcaag ctgcccatcc 2940
   agaaggaaac ctgggaaacc tggtggacag agtattggca ggccacctgg attcctgagt   3000
   gggagttcgt caacacccct cccctggtga agctgtggta ccagctggag aaggagccca   3060
   tagtgggcgc cgaaaccttc tacgtggatg gggccgctaa cagggagact aagctgggca 3120
   aagccggata cgtcactaac cggggcagac agaaggttgt caccctcact gacaccacca 3180
   accagaagac tgagctgcag gccatttacc tcgctttgca ggactcgggc ctggaggtga 3240
   acatcgtgac agactctcag tatgccctgg gcatcattca agcccagcca gaccagagtg 3300
   agtccgagct ggtcaatcag atcatcgagc agctgatcaa gaaggaaaag gtctatctgg 3360
   cctgggtacc cgcccacaaa ggcattggcg gcaatgagca ggtcgacaag ctggtctcgg 3420
   ctggcatcag gaaggtgcta ttcctggatg gcatcgacaa ggcccaggac gagcacgaga 3480
   aataccacag caactggcgg gccatggcta gcgacttcaa cctgccccct gtggtggcca 3540
   aagagatcgt ggccagctgt gacaagtgtc agctcaaggg cgaagccatg catggccagg 3600
   tggactgtag ccccggcatc tggcaactcg attgcaccca tctggagggc aaggttatcc 3660
   tggtagccgt ccatgtggcc agtggctaca tcgaggccga ggtcattccc gccgaaacag 3720
   ggcaggagac agcctacttc ctcctgaagc tggcaggccg gtggccagtg aagaccatcc 3780
   atactgacaa tggcagcaat ttcaccagtg ctacggttaa ggccgcctgc tggtgggcgg 3840
   gaatcaagca ggagttcggg atcccctaca atccccagag tcagggcgtc gtcgagtcta 3900
   tgaataagga gttaaagaag attatcggcc aggtcagaga tcaggctgag catctcaaga 3960
   ccgcggtcca aatggcggta ttcatccaca atttcaagcg gaaggggggg attggggggt 4020
   acagtgcggg ggagcggatc gtggacatca tcgcgaccga catccagact aaggagctgc 4080
   aaaagcagat taccaagatt cagaatttcc gggtctacta cagggacagc agaaatcccc 4140
   tctggaaagg cccagcgaag ctcctctgga agggtgaggg ggcagtagtg atccaggata 4200
   atagcgacat caaggtggtg cccagaagaa aggcgaagat cattagggat tatggcaaac 4260
   agatggcggg tgatgattgc gtggcgagca gacaggatga ggattag 4307
<210> 3
   <211> 2571
   <212> DNA
   <213> Human Immunodeficiency virus type I
<400> 3
   atgagagtga aggggatcag gaggaattat cagcactggt ggggatgggg cacgatgctc 60
   cttgggttat taatgatctg tagtgctaca gaaaaattgt gggtcacagt ctattatggg 120
   gtacctgtgt ggaaagaagc aaccaccact ctattttgtg catcagatgc taaagcatat 180
   gatacagagg tacataatgt ttgggccaca caagcctgtg tacccacaga ccccaaccca 240
   caagaagtag aattggtaaa tgtgacagaa aattttaaca tgtggaaaaa taacatggta 300
   gaacagatgc atgaggatat aatcagttta tgggatcaaa gcctaaagcc atgtgtaaaa 360
   ttaaccccac tctgtgttac tttaaattgc actgatttga ggaatactac taataccaat 420
   aatagtactg ctaataacaa tagtaatagc gagggaacaa taaagggagg agaaatgaaa 480
   aactgctctt tcaatatcac cacaagcata agagataaga tgcagaaaga atatgcactt 540
   ctttataaac ttgatatagt atcaatagat aatgatagta ccagctatag gttgataagt 600
   tgtaatacct cagtcattac acaagcttgt ccaaagatat cctttgagcc aattcccata 660
   cactattgtg ccccggctgg ttttgcgatt ctaaaatgta acgataaaaa gttcagtgga 720
   aaaggatcat gtaaaaatgt cagcacagta caatgtacac atggaattag gccagtagta 780
   tcaactcaac tgctgttaaa tggcagtcta gcagaagaag aggtagtaat tagatctgag 840
   aatttcactg ataatgctaa aaccatcata gtacatctga atgaatctgt acaaattaat 900
   tgtacaagac ccaactacaa taaaagaaaa aggatacata taggaccagg gagagcattt 960
   tatacaacaa aaaatataat aggaactata agacaagcac attgtaacat tagtagagca 1020
   aaatggaatg acactttaag acagatagtt agcaaattaa aagaacaatt taagaataaa 1080
   acaatagtct ttaatcaatc ctcaggaggg gacccagaaa ttgtaatgca cagttttaat 1140
   tgtggagggg aatttttcta ctgtaataca tcaccactgt ttaatagtac ttggaatggt 1200
   aataatactt ggaataatac tacaggtcaa aataacaata tcacacttca atgcaaaata 1260
   aaacaaatta taaacatgtg gcaggaagta ggaaaagcaa tgtatgcccc tcccattgaa 1320
   ggacaaatta gatgttcatc aaatattaca gggctactat taacaagaga tggtggtaag 1380
   gacacggaca cgaacgacac cgagatcttc agacctggag gaggagatat gagggacaat 1440
   tggagaagtg aattatataa atataaagta gtaacaattg aaccattagg agtagcaccc 1500
   accaaggcaa agagaagagt ggtgcagaga gaaaaaagag cagcgatagg agctctgttc 1560
   cttgggttct taggagcagc aggaagcact atgggcgcag cgtcagtgac gctgacggta 1620
   caggccagac tattattgtc tggtatagtg caacagcaga acaatttgct gagggccatt 1680
   gaggcgcaac agcatatgtt gcaactcaca gtctggggca tcaagcagct ccaggcaaga 1740
   gtcctggctg tggaaagata cctaaaggat caacagctcc tggggttttg gggttgctct 1800
   ggaaaactca tttgcaccac tactgtgcct tggaatgcta gttggagtaa taaatctctg 1860
   gatgatattt ggaataacat gacctggatg cagtgggaaa gagaaattga caattacaca 1920
   agcttaatat actcattact agaaaaatcg caaacccaac aagaaaagaa tgaacaagaa 1980
   ttattggaat tggataaatg ggcaagtttg tggaattggt ttgacataac aaattggctg 2040
   tggtatataa aaatattcat aatgatagta ggaggcttgg taggtttaag aatagttttt 2100
   gctgtacttt ctatagtgaa tagagttagg cagggatact caccattgtc gttgcagacc 2160
   cgccccccag ttccgagggg acccgacagg cccgaaggaa tcgaagaaga aggtggagag 2220
   agagacagag acacatccgg tcgattagtg catggattct tagcaattat ctgggtcgac 2280
   ctgcggagcc tgttcctctt cagctaccac cacagagact tactcttgat tgcagcgagg 2340
   attgtggaac ttctgggacg cagggggtgg gaagtcctca aatattggtg gaatctccta 2400
   cagtattgga gtcaggaact aaagagtagt gctgttagct tgcttaatgc cacagctata 2460
   gcagtagctg aggggacaga tagggttata gaagtactgc aaagagctgg tagagctatt 2520
   ctccacatac ctacaagaat aagacagggc ttggaaaggg ctttgctata a 2571
<210> 4
   <211> 2571
   <212> DNA
   <213> Artificial Sequence
<220>
   <223> Codon optimised env sequence
<400> 4
   atgagggtga aggggatccg ccgcaactac cagcactggt ggggctgggg cacgatgctc   60
   ctggggctgc tgatgatctg cagcgccacc gagaagctgt gggtgaccgt gtactacggc  120
   gtgcccgtgt ggaaggaggc caccaccacc ctgttctgcg ccagcgacgc caaggcgtac  180
   gacaccgagg tgcacaacgt gtgggccacc caggcgtgcg tgcccaccga ccccaacccc  240
   caggaggtgg agctcgtgaa cgtgaccgag aacttcaaca tgtggaagaa caacatggtg  300
   gagcagatgc atgaggacat catcagcctg tgggaccaga gcctgaagcc ctgcgtgaag  360
   ctgacccccc tgtgcgtgac cctgaactgc accgacctga ggaacaccac caacaccaac  420
   aacagcaccg ccaacaacaa cagcaacagc gagggcacca tcaagggcgg cgagatgaag  480
   aactgcagct tcaacatcac caccagcatc cgcgacaaga tgcagaagga gtacgccctg  540
   ctgtacaagc tggatatcgt gagcatcgac aacgacagca ccagctaccg cctgatctcc  600
   tgcaacacca gcgtgatcac ccaggcctgc cccaagatca gcttcgagcc catccccatc  660
   cactactgcg cccccgccgg cttcgccatc ctgaagtgca acgacaagaa gttcagcggc  720
   aagggcagct gcaagaacgt gagcaccgtg cagtgcaccc acggcatccg gccggtggtg  780
   agcacccagc tcctgctgaa cggcagcctg gccgaggagg aggtggtgat ccgcagcgag  840
   aacttcaccg acaacgccaa gaccatcatc gtgcacctga atgagagcgt gcagatcaac  900
   tgcacgcgtc ccaactacaa caagcgcaag cgcatccaca tcggccccgg gcgcgccttc  960
   tacaccacca agaacatcat cggcaccatc cgccaggccc actgcaacat ctctagagcc 1020
   aagtggaacg acaccctgcg ccagatcgtg agcaagctga aggagcagtt caagaacaag 1080
   accatcgtgt tcaaccagag cagcggcggc gaccccgaga tcgtgatgca cagcttcaac 1140
   tgcggcggcg aattcttcta ctgcaacacc agccccctgt tcaacagcac ctggaacggc 1200
   aacaacacct ggaacaacac caccggcagc aacaacaata ttaccctcca gtgcaagatc 1260
   aagcagatca tcaacatgtg gcaggaggtg ggcaaggcca tgtacgcccc ccccatcgag 1320
   ggccagatcc ggtgcagcag caacatcacc ggtctgctgc tgacccgcga cggcggcaag 1380
   gacaccgaca ccaacgacac cgaaatcttc cgccccggcg gcggcgacat gcgcgacaac 1440
   tggagatctg agctgtacaa gtacaaggtg gtgacgatcg agcccctggg cgtggccccc 1500
   accaaggcca agcgccgcgt ggtgcagcgc gagaagcggg ccgccatcgg cgccctgttc 1560
   ctgggcttcc tgggggcggc gggcagcacc atgggggccg ccagcgtgac cctgaccgtg 1620
   caggcccgcc tgctcctgag cggcatcgtg cagcagcaga acaacctcct ccgcgccatc 1680
   gaggcccagc agcatatgct ccagctcacc gtgtggggca tcaagcagct ccaggcccgc 1740
   gtgctggccg tggagcgcta cctgaaggac cagcagctcc tgggcttctg gggctgctcc 1800
   ggcaagctga tctgcaccac cacggtaccc tggaacgcct cctggagcaa caagagcctg 1860
   gacgacatct ggaacaacat gacctggatg cagtgggagc gcgagatcga taactacacc 1920
   agcctgatct acagcctgct ggagaagagc cagacccagc aggagaagaa cgagcaggag 1980
   ctgctggagc tggacaagtg ggcgagcctg tggaactggt tcgacatcac caactggctg 2040
   tggtacatca aaatcttcat catgattgtg ggcggcctgg tgggcctccg catcgtgttc 2100
   gccgtgctga gcatcgtgaa ccgcgtgcgc cagggctaca gccccctgag cctccagacc 2160
   cggccccccg tgccgcgcgg gcccgaccgc cccgagggca tcgaggagga gggcggcgag 2220
   cgcgaccgcg acaccagcgg caggctcgtg cacggcttcc tggcgatcat ctgggtcgac 2280
   ctccgcagcc tgttcctgtt cagctaccac caccgcgacc tgctgctgat cgccgcccgc 2340
   atcgtggaac tcctaggccg ccgcggctgg gaggtgctga agtactggtg gaacctcctc 2400
   cagtattgga gccaggagct gaagtccagc gccgtgagcc tgctgaacgc caccgccatc 2460
   gccgtggccg agggcaccga ccgcgtgatc gaggtgctcc agagggccgg gagggcgatc 2520
   ctgcacatcc ccacccgcat ccgccagggg ctcgagaggg cgctgctgta a          2571
<210> 5
   <211> 22
   <212> RNA
   <213> Artificial Sequence
<220>
   <223> Catalytic domain of general ribozyme (Reference Example 1)
<400> 5
   cugaugaggc cgaaaggccg aa 22
<210> 6
   <211> 22
   <212> RNA
   <213> Human immunodeficiency virus type I
<400> 6
   uaguaagaau guauagcccu ac 22
<210> 7
   <211> 22
   <212> RNA
   <213> Human immunodeficiency virus type I
<400> 7
   aacccagauu guaagacuau uu 22
<210> 8
   <211> 22
   <212> RNA
   <213> Human immunodeficiency virus type I
<400> 8
   uguuucaauu guggcaaaga ag 22
<210> 9
   <211> 22
   <212> RNA
   <213> Human immunodeficiency virus type I
<400> 9
   aaaaagggcu guuggaaaug ug 22
<210> 10
   <211> 22
   <212> RNA
   <213> Human immunodeficiency virus type I
<400> 10
   acgaccccuc gucacaauaa ag 22
<210> 11
   <211> 22
   <212> RNA
   <213> Human immunodeficiency virus type I
<400> 11
   ggaauuggag guuuuaucaa ag 22
<210> 12
   <211> 22
   <212> RNA
   <213> Human immunodeficiency virus type I
<400> 12
   auauuuuuca guucccuuag au 22
<210> 13
   <211> 22
   <212> RNA
   <213> Human immunodeficiency virus type I
<400> 13
   uggaugauuu guauguagga uc 22
<210> 14
   <211> 22
   <212> RNA
   <213> Human immunodeficiency virus type I
<400> 14
   cuuuggaugg guuaugaacu cc 22
<210> 15
   <211> 22
   <212> RNA
   <213> Human immunodeficiency virus type I
<400> 15
   cagcuggacu gucaaugaca ua 22
<210> 16
   <211> 22
   <212> RNA
   <213> Human immunodeficiency virus type I
<400> 16
   aacuuucuau guagaugggg ca 22
<210> 17
   <211> 22
   <212> RNA
   <213> Human immunodeficiency virus type I
<400> 17
   aaggccgccu guuggugggc ag 22
<210> 18
   <211> 22
   <212> RNA
   <213> Human immunodeficiency virus type I
<400> 18
   uaagacagca guacaaaugg ca 22
<210> 19
   <211> 30
   <212> DNA
   <213> Artificial Sequence
<220>
   <223> 5' PCR primer (Reference Example 1)
<400> 19
   cagctgctcg agcagctgaa gcttgeatgc 30
<210> 20
   <211> 34
   <212> DNA
   <213> Artificial Sequence
<220>
   <223> 3' PCR primer (Reference Example 1)
<400> 20
   gtaagttatg taacggacga tatcttgtct tctt 34
<210> 21
   <211> 37
   <212> DNA
   <213> Artificial Sequence
<220>
   <223> 3' PCR primer (Reference Example 1)
<400> 21
   cgcatagtcg acgggcccgc cactgctaga gattttc 37
<210> 22
   <211> 34
   <212> RNA
   <213> Hepatitis B virus
<400> 22
   ggcucgaacu ugucgugguu aucguggaug uguc 34
<210> 23
   <211> 63
   <212> RNA
   <213> Artificial Sequence
<220>
   <223> External Guide Sequence (Figure 13A)
<400> 23
   cgauagcaga cucuaaaucu gccgucaucg acuucgaagg uucgaauccu ucccaggaca 60
   cca 63
<210> 24
   <211> 23
   <212> RNA
   <213> Unknown
<220>
   <223> Target RNA sequence (Figure 13B)
<220>
   <221> misc_RNA
   <222> (1-7, 9-14, 16-23)
   <223> N-A or G or C or U
<400> 24
   nnnnnnngnn nnnnunnnnn nnn 23
<210> 25
   <211> 66
   <212> RNA
   <213> Artificial Sequence
<220>
   <223> External Guide Sequence (Figure 13B)
<220>
   <221> misc_RNA
   <222> (1-7, 56-61)
   <223> N= A or G or C or U
<400> 25
   nnnnnnnagc agacucuaaa ucugccguca ucgacuucga agguucgaau ccuucnnnnn 60
   ncacca 66
<210> 26
   <211> 49
   <212> RNA
   <213> Artificial Sequence
<220>
   <223> External Guide Sequence (Figure 13B)
<220>
   <221> misc_RNA
   <222> (1-7, 39-44)
   <223> N=A or G or C or U
<400> 26
   nnnnnnnacg ucaucgacuu cgaagguucg aauccuucnn nnnncacca 49